(19) Europäisches Patentamt
European Patent Office
Office européen des brevets

(11) EP 1 516 189 B1

(12) **EUROPEAN PATENT SPECIFICATION**

(45) Date of publication and mention of the grant of the patent:
**21.11.2007 Bulletin 2007/47**

(21) Application number: **03761509.3**

(22) Date of filing: **26.06.2003**

(51) Int Cl.:
***G01N 33/68*** *(2006.01)* ***C07K 14/47*** *(2006.01)*
***G01N 33/50*** *(2006.01)*

(86) International application number:
**PCT/EP2003/006719**

(87) International publication number:
**WO 2004/003563 (08.01.2004 Gazette 2004/02)**

(54) **DIAGNOSTIC AND THERAPEUTIC USE OF ENSADIN-0581 GENE AND PROTEIN FOR NEURODEGENERATIVE DISEASES**

DIAGNOSTISCHE UND THERAPEUTISCHE VERWENDUNG VON ENSADIN-0581 GEN UND PROTEIN FÜR NEURODEGENERATIVE ERKRANKUNGEN

UTILISATION DIAGNOSTIQUE ET THERAPEUTIQUE DE LA PROTEINE ET DU GENE ENSADIN-0581 DANS LE CADRE DES MALADIES NEURODEGENERATIVES

(84) Designated Contracting States:
**AT BE BG CH CY CZ DE DK EE ES FI FR GB GR HU IE IT LI LU MC NL PT RO SE SI SK TR**

(30) Priority: **27.06.2002 EP 02014313**
**27.06.2002 US 391614 P**

(43) Date of publication of application:
**23.03.2005 Bulletin 2005/12**

(73) Proprietor: **EVOTEC Neurosciences GmbH**
**22525 Hamburg (DE)**

(72) Inventors:
- **VON DER KAMMER, Heinz**
  **22607 Hamburg (DE)**
- **POHLNER, Johannes**
  **22175 Hamburg (DE)**
- **HANES, Jozef**
  **84107 Bratislava (SK)**

(74) Representative: **von Kreisler Selting Werner Patent Attorneys**
**Postfach 10 22 41**
**50462 Köln (DE)**

(56) References cited:
- **DATABASE EMBL [Online] 1 December 2001 (2001-12-01) "Hypothetical protein KIAA1900" retrieved from EBI Database accession no. q96py7 XP002264243**
- **NEMES JOZSEF P ET AL: "The SCA8 transcript is an antisense RNA to a brain-specific transcript encoding a novel actin-binding protein (KLHL1)" HUMAN MOLECULAR GENETICS, vol. 9, no. 10, 12 June 2000 (2000-06-12), pages 1543-1551, XP002264242 ISSN: 0964-6906**
- **BOMONT PASCALE ET AL: "The gene encoding gigaxonin, a new member of the cytoskeletal BTB/kelch repeat family, is mutated in giant axonal neuropathy" NATURE GENETICS, vol. 26, no. 3, November 2000 (2000-11), pages 370-373, XP001156497 ISSN: 1061-4036**
- **XUE FEIYU ET AL: "Kelch Encodes a component of intercellular bridges in Drosophila egg chambers" CELL, MIT PRESS, CAMBRIDGE, MA,, US, vol. 72, no. 5, 1993, pages 681-693, XP002146072 ISSN: 0092-8674 cited in the application**
- **NAGASE T ET AL: "PREDICTION OF THE CODING SEQUENCES OF UNIDENTIFIED HUMAN GENES XXI. THE COMPLETE SEQUENCES OF 60 NEW CDNA CLONES FROM BRAIN WHICH CODE FOR LARGE PROTEINS" DNA RESEARCH, UNIVERSAL ACADEMY PRESS, JP, vol. 8, no. 4, 31 August 2001 (2001-08-31), pages 179-187, XP001055485 ISSN: 1340-2838**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 516 189 B1

## Description

The present invention relates to methods of diagnosing, prognosticating, and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376).
AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).
The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). The polymorphic plasmaprotein ApoE plays a role in the intercellular cholesterol and phospholipid transport by binding low-density lipoprotein receptors, and it seems to play a role in neurite growth and regeneration. Efforts to detect further susceptibility genes and disease-linked polymorphisms, lead to the assumption that specific regions and genes on human chromosomes 10 and 12 may be associated with late-onset AD (Myers et al., Science 2000, 290: 2304-5; Bertram et al., Science 2000, 290: 2303; Scott et al., Am J Hum Genet 2000, 66: 922-32). Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The mutations found to date account for only half of the familial AD cases, which is less than 2% of all AD patients. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

The present invention discloses the differential expression of a gene coding for Ensadin-0581 in human Alzheimer's disease brain samples by using suppressive subtractive hybridization and screening of DNA biochips. The gene coding for Ensadin-0581 was originally isolated and cloned from a human fetal brain cDNA library (Genbank accession number AK055292). The human cDNA of Ensadin-0581 is highly homologous to a large human consensus sequence deduced and assembled from the sequences of Genbank entries AK055292 and AB067487 (genomic sequences AL033375, AL356958, AL023656). The human Ensadin-0581 gene harbors a predicted open reading frame coding for a protein of 620 amino acids in length, SEQ ID NO. 1, with an approximate molecular weight of about 70 kDa. The predicted protein is identical to the protein product of Genbank ID number BAB70899.1, BAB67793 (protein ID Q96NJ5), which is deduced from the sequence Genbank accession number AK055292. The Ensadin-0581 protein contains 38 additional amino acids at its N-terminus in comparison to the protein of Genbank ID number BAB67793.1, which is deduced from a cDNA with Genbank accession number AB067487. Human Ensadin-0581 protein shares weak amino acid sequence identity to the protein products of cDNAs "diablo" (dbo) (~ 30% identity) and "kelch" (~ 25% identity) of the fruitfly *Drosophila melanogaster.* No homology or similarity to any other known proteins have been identified to date. On the basis of radiation hybrid mapping studies, the Ensadin-0581 gene could be located on human chromosome 6 (Nagase et al., DNA Research 2001, 8:179-187).

Ensadin-0581 contains consensus motifs also found in other known proteins. At its N-terminus (region of amino acids 32 to 171) the Ensadin-0581 protein possesses a BTB/POZ (Bric-a-brac, Tramtrack, Broad-complex/Pox virus Zinc finger) domain-like structure (He at al., Int Rev Cytol 1995, 162B:1-74), which is one of the most conserved protein domains in the human genome. More than 100 distinct BTB/POZ containing proteins are described to date (Collins et al., Molecular and Cellular Biology 2001, 21:3609-3615). Mostly, said BTB/POZ domain has been identified in zinc finger transcription factors, but it has also been identified in other proteins, e.g. in some actin binding proteins. This domain has been implicated in protein-protein interactions. The domain is a 120 amino acid motif which by itself forms a dimer and mediates dimerization and oligomerization in proteins, thus playing a role in the assembly of large protein complexes. Additionally, it is associated with gene repression activities and is implicated in controlling gene expression. The BTB/POZ motif is located at the N-terminus of proteins and occurs together with other domains, such as, for example, kelch-, zinc finger-, or GTPase motifs, which are found more C-terminally. This also applies to Ensadin-0581 which possesses homology to "kelch motifs" at its C-terminus (region of amino acids 335 to 533). At least four of such 'kelch-like' motifs have been identified. 'Kelch' domains contain a predicted structure of four-stranded beta-sheet repeats that, according to structural analysis form so called "superbarrels" or "beta-propellers". Normally four to seven of these motifs, each 44-56 amino acids in length with eight conserved key residues, form a "kelch" domain (Adams et al., Trends in Cell Biology 2000, 10:17-24). The precise role of these repeats is not yet known, but they may function in protein folding, in binding of actin, and in protein-protein-interaction.

The kelch domain was originally identified as a sixfold tandem element in the "Kelch ORF1" protein of *Drosophila melanogaster* (Xue and Cooley, Cell 1993, 72:681-693). The "Kelch" protein is required for oocyte maturation and the production of viable eggs, as it is a component of the ring canals, which form intracellular bridges between the nurse cells and the oocyte, and thus is responsible for the transport of cytoplasma between both cell types (Robinson and Cooley, Journal of Cell Biology 1997, 138:799-810). The kelch motif is shared among several genes, some but not all belonging to the Kelch family of genes with five defined subgroups (Adams et al., Trends in Cell Biology 2000, 10:17-24). Some Kelch family proteins have been associated with Spinocerebellar ataxia-8 (SCA8) (Nemes et al., Human Molecular Genetics 2000, 9:1543-1551), and mutant forms of these proteins lead, inter alia, to deterioration of behavior and body control in animal models.

On the cellular level, kelch family proteins are widely distributed, including extracellular milieu, intracellular compartments, and the cell surface. Thus, kelch-like motif containing proteins are expected to participate in many cell functions. For instance, several actin-binding proteins contain "kelch-like" repeats. Some of them are involved in the organization of the actin cytoskeleton in brain cells. Proteins mainly localized in the brain and harboring the aforementioned properties are, for instance, NRP/B (for nuclear matrix protein expressed in brain, mayven, and actinfilin (Kim et al., Journal of Cell Biology 1998, 141:553-566; Soltysik-Espanola et al., Mol Biol Cell 1999, 10:2361-2375; Chen et al., Journal of Biological Chemistry 2002, published as manuscript M202076200). The actin-based cytoskeleton provides not only a structural framework for cells, but also does it regulate transport and distribution of proteins, whole organelles, and mRNA. Furthermore, it regulates secretional events and is involved in the formation of axons and dendrites in neuronal tissues. Thus, proteins expressed in the brain and containing a 'kelch'-motif, might be relevant for the formation of long-term memory and for maintaining neuronal function. As disclosed in the present invention, the Ensadin-0581 gene was found to be expressed in human adult brain. A highly homologous gene (Genbank accession number AB067487) was found to be expressed predominantly in human fetal and adult brain, with modest expression in other tissues such as kidney and ovary. In the human adult central nervous system, expression was particularly prominent in the hippocampus, amygdala, and cerebellum, corpus callosum, substantia nigra, thalamus, spinal cord, and in the caudate- and subthalamic nucleus (Nagase et al., DNA Research 2001, 8:179-187).

The present invention discloses a dysregulation of Ensadin-0581 gene expression on the transcriptional level in the temporal cortex region relative to the frontal lobe region of brain samples taken from AD patients. No such dysregulation is observed in samples derived from age-matched, healthy controls. To date, no experiments have been described that demonstrate a relationship between a dysregulation of Ensadin-0581 gene expression and the pathology of neurodegenerative diseases, in particular AD. Such a link, as disclosed in the present invention, offers new ways, inter alia, for the diagnosis and treatment of said diseases.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore the term "variant" shall include any shorter or longer version of a polypeptide or protein. "Variants" shall also comprise a sequence that has at least 95% sequence identity with the amino acid sequences of SEQ ID NO.1. "Variants" of a protein molecule shown in SEQ ID NO. 1 include, for example, proteins with conservative amino acid substitutions in highly conservative regions. "Proteins and polypeptides" of the present invention include variants, fragments and chemical derivatives of the protein comprising SEQ ID NO. 1. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998).

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, age-related macular degeneration, narcolepsy, motor neuron diseases, prion diseases, traumatic nerve injury and repair, and multiple sclerosis.

In one aspect, the invention features a method of diagnosing or prognosticating a neurodegenerative disease in a subject, or determining whether a subject is at increased risk of developing said disease. The method comprises: determining a level, or an activity, or both said level and said activity of (i) a transcription product of a gene coding for Ensadin-0581, and/or of (ii) a translation product of a gene coding for Ensadin-0581, and/or of (iii) a fragment, or derivative, or variant of said transcription or translation product in a sample from said subject and comparing said level, and/or said activity to a reference value representing a known disease or health status, thereby diagnosing or prognosticating said neurodegenerative disease in said subject, or determining whether said subject is at increased risk of developing said neurodegenerative disease.

The invention also relates to the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimers disease. This feature has utility for developing rapid DNA-based diagnostic tests, preferably also in the format of a kit.

In a further aspect, the invention features a method of monitoring the progression of a neurodegenerative disease in a subject. A level, or an activity, or both said level and said activity, of (i) a transcription product of a gene coding for Ensadin-0581, and/or of (ii) a translation product of a gene coding for Ensadin-0581, and/or of (iii) a fragment, or derivative, or variant of said transcription or translation product in a sample from said subject is determined. Said level and/or said activity is compared to a reference value representing a known disease or health status. Thereby the progression of said neurodegenerative disease in said subject is monitored.

In still a further aspect, the invention features a method of evaluating a treatment for a neurodegenerative disease, comprising determining a level, or an activity, or both said level and said activity of (i) a transcription product of a gene coding for Ensadin-0581, and/or of (ii) a translation product of a gene coding for Ensadin-0581, and/or of (iii) a fragment, or derivative, or variant of said transcription or translation product in a sample obtained from a subject being treated for said disease. Said level, or said activity, or both said level and said activity are compared to a reference value representing a known disease or health status, thereby evaluating the treatment for said neurodegenerative disease.

In a preferred embodiment of the herein claimed methods, kits, recombinant non-human animals, molecules, assays, and uses of the instant invention, said gene coding for Ensadin-0581 protein is the gene as shown in SEQ ID NO. 2, or fragments, derivatives, or variants thereof. Likewise, said Ensadin-0581 protein is the protein as shown in SEQ ID NO. 1, or fragments, derivatives, or variants thereof.

In a further preferred embodiment of the herein claimed methods, kits, recombinant animals, molecules, assays, and uses of the instant invention, said neurodegenerative disease or disorder is Alzheimer's disease, and said subjects suffer from Alzheimer's disease.

The present invention discloses the detection and differential expression and regulation of a gene coding for Ensadin-0581 in specific brain regions of AD patients. Consequently, the Ensadin-0581 gene and its corresponding transcription and/or translation products may have a causative role in the regional selective neuronal degeneration typically observed in AD. Alternatively, Ensadin-0581 may confer a neuroprotective function to the remaining surviving nerve cells. Based on these disclosures, the present invention has utility for the diagnostic evaluation and prognosis as well as for the identification of a predisposition to a neurodegenerative disease, in particular AD. Furthermore, the present invention provides methods for the diagnostic monitoring of patients undergoing treatment for such a disease.

It is particularly preferred that said sample to be analyzed and determined is selected from the group comprising brain tissue, or other tissues, or body cells. The sample can also comprise cerebrospinal fluid or other body fluids including saliva, urine, blood, serum plasma, or mucus. Preferably, the methods of diagnosis, prognosis, monitoring the progression or evaluating a treatment for a neurodegenerative disease, according to the instant invention, can be practiced ex *corpore,* and such methods preferably relate to samples, for instance, body fluids or cells, removed, collected, or isolated from a subject or patient.

In further preferred embodiments, said reference value is that of a level, or an activity, or both said level and said activity of (i) a transcription product of a gene coding for Ensadin-0581, and/or of (ii) a translation product of a gene coding for Ensadin-0581, and/or of (iii) a fragment, or derivative, or variant of said transcription or translation product in a sample from a subject not suffering from said neurodegenerative disease.

In preferred embodiments, an alteration in the level and/or activity of a transcription product of a gene coding for Ensadin-0581 and/or a translation product of a gene coding for Ensadin-0581 and/or a fragment, or derivative, or variant thereof in a sample cell, or tissue, or body fluid from said subject relative to a reference value representing a known health status indicates a diagnosis, or prognosis, or increased risk of becoming diseased with a neurodegenerative disease, particularly AD.

In preferred embodiments, measurement of the level of transcription products of an Ensadin-0581 gene is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of an Ensadin-0581 gene and/or a fragment, or derivative, or variant of said translation product, and/or a level of activity of said translation product, and/or a fragment, or derivative, or variant thereof, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots, and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In a preferred embodiment, the level, or the activity, or both said level and said activity of (i) a transcription product of a gene coding for Ensadin-0581, and/or of (ii) a translation product of a gene coding for Ensadin-0581, and/or of (iii) a fragment, or derivative, or variant of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features a kit for diagnosing or prognosticating neurodegenerative diseases, in particular AD, in a subject, or determining the propensity or predisposition of a subject to develop a neurodegenerative disease, in particular AD, said kit comprising:

(a) at least one reagent which is selected from the group consisting of (i) reagents that selectively detect a transcription product of a gene coding for Ensadin-0581, and (ii) reagents that selectively detect a translation product of a gene coding for Ensadin-0581; and

(b) an instruction for diagnosing or prognosticating a neurodegenerative disease, in particular AD, or determining the propensity or predisposition of a subject to develop such a disease by

- detecting a level, or an activity, or both said level and said activity, of said transcription product and/or said translation product of a gene coding for Ensadin-0581, in a sample from said subject; and
- diagnosing or prognosticating a neurodegenerative disease, in particular AD, or determining the propensity or predisposition of said subject to develop such a disease,

wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status, indicates a diagnosis or prognosis of a neurodegenerative disease, in particular AD, or an increased propensity or predisposition of developing such a disease. The kit, according to the present invention, may be particularly useful for the identification of individuals that are at risk of developing a neurodegenerative disease, in particular AD. Consequently, the kit, according to the invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of a neurodegenerative disease, in particular AD, in a subject, as well as monitoring success or failure of therapeutic treatment for such a disease of said subject.

In a further aspect, the invention features a recombinant, non-human animal comprising a non-native gene sequence coding for Ensadin-0581, or a fragment, or derivative, or variant thereof. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of neuropathology similar to a neurodegenerative disease, in particular AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). It is preferred to make use of such a recombinant non-human animal as an animal model for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease.

In another aspect, the invention features an assay for screening for a modulator of neurodegenerative diseases, in particular AD, or related diseases and disorders of one or more substances selected from the group consisting of (i) a gene coding for Ensadin-0581, and/or (ii) a transcription product of a gene coding for Ensadin-0581, and/or (iii) a translation product of a gene coding for Ensadin-0581, and/or (iv) a fragment, or derivative, or variant of (i) to (iii). This screening method comprises (a) contacting a cell with a test compound, and (b) measuring the activity, or the level, or both the activity and the level of one or more substances recited in (i) to (iv), and (c) measuring the activity, or the level, or both the activity and the level of said substances in a control cell not contacted with said test compound, and (d) comparing the levels of the substance in the cells of step (b) and (c), wherein an alteration in the activity and/or level of said substances in the contacted cells indicates that the test compound is a modulator of said diseases and disorders.

In one further aspect, the invention features a screening assay for a modulator of neurodegenerative diseases, in particular AD, or related diseases and disorders of one or more substances selected from the group consisting of (i) a gene coding for Ensadin-0581, and/or (ii) a transcription product of a gene coding for Ensadin-0581, and/or (iii) a translation product of a gene coding for Ensadin-0581, and/or (iv) a fragment, or derivative, or variant of (i) to (iii), comprising (a) administering a test compound to a test animal which is predisposed to developing or has already developed symptoms of a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed said symptoms and to which animal no such test compound has been administered, and (d) comparing the activity and/or level of the substance in the animals of step (b) and (c), wherein an alteration in the activity and/or level of substances in the test animal indicates that the test compound is a modulator of said diseases and disorders.

In a preferred embodiment, said test animal and/or said control animal is a recombinant, non-human animal which expresses a gene coding for Ensadin-0581, or a fragment, or derivative, or variant thereof, under the control of a transcriptional regulatory element which is not the native Ensadin-0581 gene transcriptional control regulatory element.

In another embodiment, the present invention provides a method for producing a medicament comprising the steps of (i) identifying a modulator of neurodegenerative diseases by a method of the aforementioned screening assays and (ii) admixing the modulator with a pharmaceutical carrier. However, said modulator may also be identifiable by other types of screening assays.

In another aspect, the present invention provides for an assay for testing a compound, preferably for screening a plurality of compounds, for inhibition of binding between a ligand and a translation product of a gene coding for Ensadin-0581, or a fragment, or derivative, or variant thereof. Said screening assay comprises the steps of (i) adding a liquid suspension of said Ensadin-0581 translation product, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said Ensadin-0581 translation product, or said fragment, or derivative, or variant thereof, and said compound or plurality of compounds, and said detectable, preferably fluorescently labelled ligand, and (v) measuring the amounts of preferably fluorescence associated with said Ensadin-0581 translation product, or with said fragment, or derivative, or variant thereof, and (vi) determining the degree of inhibition by one or more of said compounds of binding of said ligand to said Ensadin-0581 translation product, or said fragment, or derivative, or variant thereof. It might be preferred to reconstitute said Ensadin-0581 translation product, or fragment, or derivative, or variant thereof into artificial liposomes to generate the corresponding proteoliposomes to determine the inhibition of binding between a ligand and said Ensadin-0581 translation product. Methods of reconstitution of Ensadin-0581 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to an Ensadin-0581 translation product, or a fragment, or derivative, or variant thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436, WO 01/59416.

In one further embodiment, the present invention provides a method for producing a medicament comprising the steps of (i) identifying a compound as an inhibitor of binding between a ligand and a gene product of the gene coding for Ensadin-0581 by the aforementioned inhibitory binding assay and (ii) admixing the compound with a pharmaceutical carrier. However, said compound may also be identifiable by other types of screening assays.

In another aspect, the invention features an assay for testing a compound, preferably for screening a plurality of compounds to determine the degree of binding of said compounds to a translation product of the gene coding for Ensadin-0581, or to a fragment, or derivative, or variant thereof. Said screening assay comprises (i) adding a liquid suspension of said Ensadin-0581 translation product, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said Ensadin-0581 translation product, or said fragment, or derivative, or variant thereof, and said detectable, preferably fluorescently labelled compound or fluorescently labelled compounds, and (iv) measuring the amounts of preferably fluorescence associated with said Ensadin-0581 translation product, or with said fragment, or derivative, or variant thereof, and (v) determining the degree of binding by one or more of said compounds to said Ensadin-0581 translation product, or said fragment, or derivative, or variant thereof. In this type of assay it might be preferred to use a fluorescent label. However, any other type of detectable label might also be employed. Said assay methods may be useful for the identification of novel compounds as well as for evaluating compounds which have been improved or otherwise optimized in their ability to bind to an Ensadin-0581 translation product, or fragment, or derivative, or variant thereof.

In one further embodiment, the present invention provides a method for producing a medicament comprising the steps of (i) identifying a compound as a binder to a gene product of the Ensadin-0581 gene by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

The present invention features a protein molecule shown in SEQ ID NO. 1, said protein molecule being a translation product of the gene coding for an Ensadin-0581 protein, or a fragment, or derivative, or variant thereof, for use as a diagnostic target for detecting a neurodegenerative disease, preferably Alzheimer's disease.

The present invention further features a protein molecule shown in SEQ ID NO.1, said protein molecule being a translation product of the gene coding for an Ensadin-0581 protein, or a fragment, or derivative, or variant thereof, for use as a screening target for reagents or compounds preventing, or treating, or ameliorating a neurodegenerative disease, preferably Alzheimer's disease.

The present invention features an antibody which is specifically immunoreactive with an immunogen, wherein said immunogen is a translation product of a gene coding for Ensadin-0581, SEQ ID NO. 1, or a fragment, or variant, or derivative thereof. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the Ensadin-0581 gene, or fragments, or derivatives, or variants thereof.

In a preferred embodiment of the present invention, said antibodies can be used for detecting a pathological state of a cell in a sample from a subject, comprising immunocytochemical staining of said cell with said antibody, wherein an altered degree of staining, or an altered staining pattern in said cell compared to a cell representing a known health status indicates a pathological state of said cell. Preferably, the pathological state relates to a neurodegenerative disease, in particular to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

Figure 1 depicts the brain regions with selective vulnerability to neuronal loss and degeneration in AD. Primarily, neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions. In contrast, neurons within the frontal cortex, the occipital cortex, and the cerebellum remain largely intact and preserved from neurodegenerative processes in AD. Brain tissues from the frontal cortex (F), the temporal cortex (T) and the hippocampus (H) of AD patients and healthy, age-matched control individuals were used for the herein disclosed examples. For illustrative purposes, the image of a normal healthy brain was taken from a publication by Strange (Brain Biochemistry and Brain Disorders, Oxford University Press, Oxford, 1992, p.4).

Figures 2 and 3 illustrate the verification of the differential expression of the human Ensadin-0581 gene in AD brain tissues by quantitative RT-PCR analysis. Quantification of RT-PCR products from RNA samples collected from the frontal cortex (F) and the temporal cortex (T) of AD patients (Figure 2a) and samples from the frontal cortex (F) and the hippocampus (H) of AD patients (Figure 3a) was Performed by the LightCycler rapid thermal cycling technique. Likewise, samples of healthy, age-matched control individuals were compared (Figure 2b for frontal cortex and temporal cortex, Figure 3b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin.

The figures depict the kinetics of amplification by plotting the cycle number against the amount of amplified material as measured by its fluorescence. Note that the amplification kinetics of Ensadin-0581 cDNAs from both, the frontal and temporal cortices of a normal control individual, and from the frontal cortex and hippocampus of a normal control individual, respectively, during the exponential phase of the reaction are juxtaposed (Figures 2b and 3b, arrowhead), whereas in Alzheimer's disease (Figures 2a and 3a, arrowhead) there is a significant separation of the corresponding curves, indicating a differential expression of the gene coding for Ensadin-0581 in the respective analyzed brain regions.

Figure 4 discloses SEQ ID NO. 1; the amino acid sequence of the Ensadin-0581 protein. The full length human Ensadin-0581 protein comprises 620 amino acids.

Figure 5 shows SEQ ID NO. 2, the nucleotide sequence of the human Ensadin-0581 cDNA, comprising 3693 nucleotides.

Figure 6 depicts SEQ ID NO. 3, the nucleotide sequence of the 328 bp Ensadin-0581 cDNA fragment, identified and obtained by suppressive subtractive hybridization on biochips and by subsequent cloning (sequence in 5' to 3' direction).

Figure 7 outlines the sequence alignment of SEQ ID NO. 3 to the consensus sequence of the nucleotide sequences GenBank accession number AK055292 and AB067487.

Figure 8 charts the schematic alignment of SEQ ID NO. 3 with Ensadin-0581 cDNA. The open rectangle represents the Ensadin-0581 open reading frame, thin bars represent the 5' and 3' untranslated regions (UTRs).

Table 1 discloses the initial identification of differential expression of the human Ensadin-0581 gene by subtractive suppressive microarray hybridization experiments. Identical biochips containing cDNA clones of subtracted AD and control brain cDNA libraries were co-hybridized with different Cyanine3 (Cy3) and Cyanine5 (Cy5) labeled cDNA probes, designated as probes A, B, or C, respectively. Cy3 and Cy5 labeled cDNA probes (A) were generated by labeling cDNAs from frontal or temporal cortex of AD patients and control persons, respectively, refer to section (vi-a) of the example description. Cy3 and Cy5 labeled SMART probes (B) were generated from cDNAs, derived from frontal or temporal cortex of AD patients and control persons, respectively, refer to section (vi-b). Cy3 and Cy5 labeled SSH probes (C) were derived from cDNA populations after suppressive subtractive hybridization of brain cDNAs from frontal and temporal cortex of AD patients and of control individuals, respectively, refer to section (vi) ($PF_{SSH(1)}$ = AD patients frontal cortex cDNA after subtraction of AD patients temporal cortex cDNA; $PT_{SSH(2)}$ = AD patients temporal cortex cDNA after subtraction of AD patients frontal cortex cDNA). The table lists the gene expression level of Ensadin-0581 indicated as the ratio of fluorescence intensity measured for the temporal cortex relative to the frontal cortex of AD patients. The ratios of fluorescence intensity reflect a differential regulation of human Ensadin-0581 RNA expression in temporal and frontal cortex of AD patients.

Table 2 lists Ensadin-0581 gene expression levels in the temporal cortex relative to the frontal cortex in seven AD patients, herein identified by internal reference numbers P010, P011, P012, P014, P016, P017, P019 (1.01 to 1.95 fold) and five healthy, age-matched control individuals, herein identified by internal reference numbers C005, C008, C011, C012, C014 (0.51 to 1.02 fold). The scatter plot diagram visualizes individual values of the temporal to frontal cortex regulation ratios in control samples (dots) and in AD patient samples (triangles), respectively. The values shown are calculated according to the formula described herein (see below).

Table 3 lists the gene expression levels in the hippocampus relative to the frontal cortex for the Ensadin-0581 gene in six Alzheimer's disease patients, herein identified by internal reference numbers P010, P011, P012, P014, P016, P019 (0.99 to 2.41 fold) and three healthy, age-matched control individuals, herein identified by internal reference numbers C004, C005, C008 (0.81 to 2.04 fold). The scatter plot diagram visualizes individual values of the hippocampus to frontal cortex regulation ratios in, control samples (dots) and in AD patient samples (triangles). The values shown are calculated according to the formula described herein (see below).

EXAMPLE I:

(i) Brain tissue dissection from patients with AD:

Brain tissues from AD patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at -80°C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were used to generate a melting curve with the LightCycler technology as described in the manufacturer's protocol (Roche).

(iii) cDNA synthesis and Rsa I digestion

In order to identify changes in gene expression in different tissues, a screening method combining cDNA synthesis, suppressive subtractive hybridization (SSH) and screening of microarray chips with a diversity of cDNA probes from SSH was employed. This technique compares different populations of mRNA and provides clones of genes that are expressed in one population of cells but not, or at lower level, in the other population of cells. In the present invention, RNA populations from selected post-mortem brain tissues (frontal and temporal cortex) of AD patients and age-matched control subjects were compared.
As starting material for the suppressive subtractive microarray analysis total RNA was extracted as described above (ii). For production of preferably full-length cDNAs, the polymerase chain reaction (PCR)-based method 'SMART cDNA Synthesis' was performed according to the manufacturer's protocol (Clontech). The principle of 'SMART cDNA synthesis' has been described in detail (Chenchik et al., in Gene Cloning and Analysis by RT-PCR, eds. Siebert and Larrick, Biotechniques Books, Natick 1998:305-320). For SMART cDNA synthesis, four RNA pools, each consisting of 8 μg total RNA, were prepared. Each pool contained 2 μg of each of four different samples, i.e. from inferior frontal cortex (CF) and from inferior temporal cortex (CT) of control brains, from inferior frontal cortex (PF) and from inferior temporal cortex (PT) of patient brains, respectively. An amount of 1 μg of total RNA mix was utilized in a reaction volume of 50 μl (PCR cycler: Multi Cycler PTC 200, MJ Research). The second SMART PCR step was performed using 19 cycles. SuperScript II RNaseH Reverse Transcriptase and 5x first-strand buffer (Invitrogen) were used.
After extraction and purification of the PCR products, restriction digestions were carried out with 30 U Rsa I (MBI Fermentas) for 2.5 hours at 37°C. Rsa I restriction sites are located within the universal priming sites of the double stranded (ds) cDNA. The quality of the digestions was analyzed by agarose gel electrophoresis, the digested samples were purified (QIAquick PCR Purification Kit, Qiagen), and the cDNA concentrations were determined by UV spectrophotometry (Biorad).

(iv) Suppressive subtractive hybridization (SSH)

Four SMART cDNA pools (iii), were compared using suppressive subtractive hybridization. A pool of cDNA containing differentially expressed genes is thereby designated as "Tester", the reference cDNA pool as "Driver". The two pools are hybridized, and all cDNAs, present in both pools, will be eliminated, i.e. the Driver-pool will be subtracted from the Tester-pool. Thus, clones of genes that are predominantly expressed in the Tester population are obtained.
The 'PCR-Select cDNA Subtraction Kit' was used to perform the subtractive hybridization (Clontech). The 'Tester' SMART cDNA pools, derived from frontal and temporal cortex (CF and CT) of control brains, and from frontal and temporal cortex (PF and PT) of patient brains (iii), were subdivided into two pools each. Each pool was ligated with Adaptor 1 or Adaptor 2, respectively, thus obtaining 6 different 'Tester' cDNA pools. The three 'Driver' SMART cDNA pools, CT, PF and PT, remained unligated. In a first hybridization step, used to enrich for differentially expressed sequences, the following three different 'Tester' SMART cDNA pools were combined with an excess of the following 'Driver' SMART cDNAs: SSH(1): PF-'Tester' and PT-'Driver'; SSH(2): PT-'Tester' and PF-'Driver'; SSH(3): CT-'Tester' and PT-'Driver'; SSH(4): PT-'Tester' and CT-'Driver'. Following a denaturation step for 1.5 min at 98°C, the hybridization was carried out for 8 hours at 68°C. In a second step, the two corresponding primary hybridization samples of 'Tester' SMART cDNA pools ligated to Adaptor 1 or 2, respectively, were mixed and re-hybridized at 68°C for 15 hours, with an excess of the 'Driver' SMART cDNA pool, as used before. Thus, suitable double stranded cDNAs for subsequent amplification, i.e. with both Adaptor sequences at their 5' and 3' ends and therefore with different annealing sites, were generated. The following PCR steps were applied to obtain efficiently amplified specific products and to suppress nonspecific amplification. In the first PCR, missing strands of the adaptors were filled in by DNA-polymerase activity. 1 μl of the obtained hybridization products each were subjected to PCR using the corresponding 'Primer 1' (10 μM) (Clontech) along with 1x PCR reaction buffer (Clontech), 10 mM dNTP-Mix (dATP, dGTP, dCTP, dTTP, Amersham Pharmacia Biotech), and 0.5 μl 50x Advantage cDNA Polymerase Mix (Clontech) in a 25 μl final volume. PCR conditions were set as follows: one round at 75 °C for 5 min, which was followed by 27 or 30 cycles: 94 °C for 30 sec, 64 °C or 66 °C for 30 sec, 72 °C for 1.5 min. One final step at 72 °C for 5 min was added to the last cycle. A second nested PCR was performed as described for the first PCR, except that instead of 'Primer 1' the nested primers 'Nested Primer 1' and '2R' were used and an annealing temperature of 66°C or 68°C and 12 or 15 cycles, were applied. PCR-products obtained by different conditions were pooled for subsequent analysis. For the primer sequences used, refer to appendix B of the supplier's user manual (Clontech).

(v) Cloning of subtracted PCR products and production of DNA-biochips

The SSH SMART double stranded cDNAs of the four different combinations SSH(1)-SSH(4), refer to (iv), were ligated into the pCR2.1-vector and transformed into INValphaF' cells according to the manufacturer's instructions (TA Cloning Kit, Invitrogen). Bacterial colonies were picked and analyzed by colony PCR on MTPs (microtiter plates, 96 well, Abgene), using 'Nested Primer 1' and 'Nested Primer 2'. Those MTPs showing more than 90% positive clones were subjected to a preparative colony PCR approach. Per well, the following PCR mix was generated: the corresponding oligonucleotides 'Nested Primer 1' and 'Nested Primer 2' (0.5 μM each), 1 x Titanium PCR buffer (Clontech), 200 μM dNTP-Mix (Amersham Pharmacia Biotech), 0.2 x TitaniumTaq DNA-Polymerase (Clontech) in a 120 μl final volume. PCR conditions were set as follows: one round at 94 °C for 30 sec for denaturing, the next round was followed by 35 cycles: 94 °C for 30 sec and 68°C for 3 min. The quality of the amplified products was checked and analyzed (DNA LabChip system, Agilent 2100 Bioanalyzer, Agilent Technologies), followed by purification (Multiscreen-PCR-Purification system, Millipore).
Additionally, the following standard control samples were generated: three different *Arabidopsis thaliana* genes, polyA-DNA, salmon sperm DNA, human Cot-1 DNA, and 3xSSC-buffer were used as negative controls (Microarray Validation System, Stratagene); beta-Actin and Xenopus cDNA were used as normalizing controls.
Several MTPs were made of each of the SSH combinations SSH(1)-(4), harboring amplification products of 96 different clones per plate. The amplified products were spotted in triplicates onto GAPS glass-slides (CMT-GAPS, Corning) by GeneScan Europe.

(vi) Probe synthesis and identification of differentially expressed genes by screening of DNA biochips

A: cDNA probe synthesis

As starting material for the generation of Cyanine3 (Cy3) and Cyanine5 (Cy5) labeled cDNA probes total RNA was extracted and used as described above (ii) and (iii). Two samples of a mix of 2 μg of total RNA and additionally 2 ng Xenopus RNA (standard RNA) per labeling reaction were subjected to a specific reverse transcriptase reaction, whereby the polyA-mRNA is converted into fluorescein-12-dCTP (FL) or biotin-11-dCTP (B) labeled cDNA. The RNA samples derived from frontal cortex (CF) of control brains were labeled with fluorescein, the RNA from the temporal cortex (PT) of patient brains with biotin, respectively. The cDNA reactions were performed according to the Micromax TSA Labeling protocol (NEN Life Science). The purified cDNA probes were resuspended in hybridization buffer and denatured for 7 min at 100°C. Subsequently, half the volume of the fluorescein-labeling reaction (i.e. 1 μg RNA) and half the amount of the biotin-probe were mixed together in 5xSSC, 0.1% SDS, 25% formamide buffer, and applied evenly onto one prehybridized (5x SSC, 0.1% SDS, 1% BSA, 45 min at 42°C) microarray. Array hybridization was performed over night at 42°C.
Following a high stringency wash step, the detection of the bound fluorescein- and biotin labeled probes was performed according to the instructions of the TSA Detection Kit protocol (NEN Life Science). Thereby, in a first step, anti-FL-HRP (fluorescein-horseradish peroxidase) binds to the FL-labeled cDNA probe, and HRP catalyzes the deposition of the fluorescent reporter Cy3 tyramide. In a second step, streptavidin-HRP binds to the B-labeled cDNA probe and catalyzes the deposition of the fluorescent reporter molecule Cy5 tyramide. Biochip 3 was hybridized with cDNA mix PF (Cy3) and PT(Cy5). Scanning the microarrays with the appropriate wavelengths (635 nm, 532 nm) allowed detection of both cyanine dyes simultaneously.

B: SMART probe synthesis

For the production of Cyanine3 (Cy3) and Cyanine5 (Cy5) labeled SMART cDNA-probes the PCR-based method 'SMART cDNA Synthesis' was performed as described in section (iii). Here we used total RNA as starting material which was extracted as described above (ii). Four RNA mixtures were prepared as described in section (iii). 1 μg of each RNA mix and 1 ng Xenopus total RNA were subjected to the SMART cDNA reaction. For PCR amplification, extraction and purification of the cDNAs, restriction digestion with Rsa I, and subsequent purification of the digested samples, refer to section (iii).
SMART cDNA samples were labeled with either Cy3 or Cy5 (Atlas Glass Fluorescent Labeling Kit, Clontech). In the first labeling step, aliphatic amino groups, i.e. aminoallyl-dUTP (Clontech), were incorporated into denatured (100°C, 7 min) Rsa I digested PCR products. The reaction was catalyzed by the Klenow Fragment (MBI Fermentas). In a second labeling step, the fluorescent reporter dyes Cy3 or Cy5 were coupled to the incorporated functionalities. The purified Cy3 and Cy5 labeled SMART cDNA probes (Atlas NucleoSpin Extraction Kit, Clontech) were resuspended in hybridization buffer (5x SSC, 0.1 % SDS, 25% formamide) after denaturation for 7 min at 100°C. Subsequently, the Cy3 labeled SMART probe was mixed with the Cy5 labeled SMART probe and together applied evenly onto one prehybridized (5x SSC, 0.1% SDS, 1% BSA, 45 min at 42°C) microarray. Array hybridization was performed over night at 42°C. High stringency washing of the biochips followed according to the instructions of the TSA Detection Kit protocol (NEN Life Science). Biochip 2 was hybridized with SMART cDNA mix PF(Cy3) and PT(Cy5). Scanning the microarrays with the appropriate wavelengths (635 nm, 532 nm) allowed detection of both cyanine dyes simultaneously.

C: Subtraction probe synthesis

For the production of Cyanine3 (Cy3) and Cyanine5 (Cy5) labeled SSH cDNA-probes, the PCR-based method 'SMART cDNA Synthesis' was performed as described in section (iii). Here we used total RNA as starting material which was extracted as described above (ii). Four RNA mixtures were prepared as disclosed in section (iii). 1 $\mu$g of each RNA mix was subjected to the SMART cDNA reaction. For PCR amplification, extraction and purification of the cDNAs, restriction digestion with Rsa I, and subsequent purification of the digested samples, refer to section (iii). For subtractive hybridization, the PCR-Select cDNA Subtraction Kit (Clontech) was utilized as described in detail in section (iv). The subtracted PCR products of the combinations SSH(1) and SSH(2), and of SSH(3) and SSH(4), respectively, were purified (StrataClean Kit, Stratagene), and Adaptor 1 and 2 removed by restriction digest with Rsa I and Sma I (MBI Fermentas). The SSH cDNA pools were labeled with either Cy3 or Cy5 (Atlas Glass Fluorescent Labeling Kit, Clontech). In the first labeling step, aliphatic amino groups, i.e. aminoallyl-dUTP (Clontech), were incorporated into the denatured (100°C, 7 min) Rsa I and Sma I digested SSH cDNA products. The reaction was catalyzed by the Klenow Fragment (MBI Fermentas). In a second labeling step, the fluorescent reporter dyes Cy3 and Cy5 were coupled to the incorporated functionalities. The purified Cy3 and Cy5 labeled SSH cDNA probes (for purification refer to the Atlas NucleoSpin Extraction Kit, Clontech) were resuspended in hybridization buffer (5x SSC, 0.1% SDS, 25% formamide) after denaturation for 7 min at 100°C. Subsequently, the Cy3 labeled SSH1 probe was mixed with the Cy5 labeled SSH2 probe, and the Cy3 labeled SSH3 probe with the Cy5 labeled SSH4 probe, respectively. Each combination was applied evenly onto one prehybridized (5x SSC, 0.1% SDS, 1% BSA, 45 min at 42°C) microarray. Array hybridization was performed over night at 42°C. High stringency washing of the biochips followed according to the instructions of the TSA Detection Kit protocol (NEN Life Science). Biochip 1 was hybridized with the cDNA mix SSH(1)(Cy3) and SSH(2)(Cy5). Scanning of the microarrays with the appropriate wavelengths (635 nm, 532 nm) allowed detection of both cyanine dyes simultaneously.

(vii) DNA biochips data evaluation

Fluorescence raw data for Cy3 and Cy5, measured at 635 and 532 nm, respectively, were taken severalfold (for each of the three spots per cDNA). One set of measurements was performed within the spot area (signal), and another set of measurements was taken nearby (background). Subsequently the net fluorescence intensity ($FI_{635}$, $FI_{532}$) of the spots was calculated as follows:

$$FI_{635/532} = (M\ FI_{spot} - 1\ SD\ FI_{spot}) - (M\ FI_{background} + 1\ SD\ FI_{background}).$$

In this calculation, M defines the median of the replicate measurements per spot, SD the standard deviation of the corresponding mean. Subsequently, only $FI_{635}$ and $FI_{532}$ values of >2 were considered for further evaluation plus those $FI_{635}$ and $FI_{532}$ values of <2 where the corresponding value for the second wavelength was >3. In an analogous manner, the corresponding values for the Xenopus cDNA control and the set of standard (housekeeping) genes were evaluated. The Xenopus cDNA was used as an internal calibrator for the efficiency of cDNA synthesis of the disease relevant mRNAs. Then, from the background corrected $FI_{635/532}$ medians of the three replicate spots, the statistical mean was calculated and the signal ratio R for the cDNA probes was derived using formula:

$$R_{635/532} = FI_{635,\ calibrated} / FI_{532,\ calibrated}.$$

In a last step of evaluation, the results of the different hybridizations were considered for logical coherence.

(viii) Confirmation of differential expression by quantitative RT-PCR:

Positive corroboration of differential expression of the gene coding for Ensadin-0581 was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint readout. The ratios of Ensadin-0581 cDNA from the temporal cortex and frontal cortex, and from the hippocampus and frontal cortex, respectively, were determined (relative quantification).

First, a standard curve was generated to determine the efficiency of the PCR with specific primers for the gene coding for Ensadin-0581 (5'-GGCACCATCTGAAAAGCCAA-3' and 5'-CCCAGTATCCAACAGTCAGAGCT-3'). PCR amplification (95 °C and 1 sec, 56 °C and 5 sec, and 72 °C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed a single peak at approximately 80°C with no visible primer dimers. Quality and size of the PCR product were determined with the DNA LabChip system (Agilent 2100 Bioanalyzer, Agilent Technologies). A single peak at the expected size of 68 bp for the Ensadin-0581 gene was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'- TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for Ensadin-0581 and the five reference standard genes. The slopes and the intercepts of the standard curves (i.e. linear regressions) were calculated for all genes. In a second step, cDNAs from temporal cortex and frontal cortex, and from hippocampus and frontal cortex, respectively, were analyzed in parallel and normalized to cyclophilin B. The $C_t$ values were measured and converted to ng total brain cDNA using the corresponding standard curves:

$$10 \wedge ((C_t \text{ value - intercept}) / \text{slope}) \quad [\text{ng total brain cDNA}]$$

The values for temporal and frontal cortex Ensadin-0581 cDNAs, and the values for hippocampus and frontal cortex Ensadin-0581 cDNAs, respectively, were normalized to cyclophilin B and the ratios were calculated according to formulas:

$$\text{Ratio} = \frac{\text{Ensadin-0581 temporal [ng] / cyclophilin B temporal [ng]}}{\text{Ensadin-0581 frontal [ng] / cyclophilin B frontal [ng]}}$$

$$\text{Ratio} = \frac{\text{Ensadin-0581 hippocampus [ng] / cyclophilin B hippocampus [ng]}}{\text{Ensadin-0581 frontal [ng] / cyclophilin B frontal [ng]}}$$

In a third step, the set of reference standard genes was analyzed in parallel to determine the mean average value of the temporal to frontal ratios, and of the hippocampal to frontal ratios, respectively, of expression levels of the reference standard genes for each individual brain sample. As cyclophilin B was analyzed in step 2 and step 3, and the ratio from one gene to another gene remained constant in different runs, it was possible to normalize the values for Ensadin-0581 to the mean average value of the set of reference standard genes instead of normalizing to one single gene alone. The calculation was performed by dividing the ratio shown above by the deviation of cyclophilin B from the mean value of all housekeeping genes. The results of such quantitative RT-PCR analysis for the Ensadin-0581 gene are shown in Figures 2 and 3.

## Claims

**1.** A method of diagnosing or prognosticating a neurodegenerative disease in a subject, or determining whether a subject is at increased risk of developing said disease, comprising:

determining a level and/or an activity of

(i) a transcription product of a gene coding for a protein having SEQ ID NO.1, and/or
(ii) a translation product of a gene coding for a protein having SEQ ID NO.1, and/or
(iii) a fragment, or derivative, or variant of said transcription or translation product, wherein the variant has at least 95% sequence identity with the amino acid sequences of SEQ ID NO.1

in a sample from said subject and comparing said level and/or said activity to a reference value representing a known disease or health status, thereby diagnosing or prognosticating said neurodegenerative disease in said subject, or determining whether said subject is at increased risk of developing said neurodegenerative disease.

**2.** The method according to claim 1 wherein said neurodegenerative disease is Alzheimer's disease.

**3.** A kit for diagnosing or prognosticating a neurodegenerative disease, in particular Alzheimer's disease, in a subject, or determining the propensity or predisposition of a subject to develop such a disease, said kit comprising:

(a) at least one reagent which is selected from the group consisting of (i) reagents that selectively detect a transcription product of a gene coding for a protein having SEQ ID NO.1 and (ii) reagents that selectively detect a translation product of a gene coding for a protein having SEQ ID NO.1; and
(b) an instruction for diagnosing or prognosticating a neurodegenerative disease, in particular Alzheimer's disease, or determining the propensity or predisposition of a subject to develop such a disease by (i) detecting a level, or an activity, or both said level and said activity, of said transcription product and/or said translation product of a gene coding for a protein having SEQ ID NO.1, in a sample from said subject; and (ii) diagnosing or prognosticating a neurodegenerative disease, in particular Alzheimer's disease, or determining the propensity or predisposition of said subject to develop such a disease, wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status indicates a diagnosis or prognosis of a neurodegenerative disease, in particular Alzheimer's disease, or an increased propensity or predisposition of developing such a disease.

**4.** A recombinant, non-human animal comprising a non-native gene sequence coding for a protein having SEQ ID NO. 1, or a fragment, or a derivative, or a variant thereof, wherein the variant has at least 95% sequence identity with the amino acid sequences of SEQ ID NO.1, said animal being obtainable by:

(i) providing a gene targeting construct comprising said gene sequence and a selectable marker sequence, and
(ii) introducing said targeting construct into a stem cell of a non-human animal, and
(iii) introducing said non-human animal stem cell into a non-human embryo, and
(iv) transplanting said embryo into a pseudopregnant non-human animal, and
(v) allowing said embryo to develop to term, and
(vi) identifying a genetically altered non-human animal whose genome comprises a modification of said gene sequence in both alleles, and
(vii) breeding the genetically altered non-human animal of step (vi) to obtain a genetically altered non-human animal whose genome comprises a modification of said endogenous gene, wherein said disruption results in said non-human animal exhibiting a predisposition to developing symptoms of a neurodegenerative disease or related diseases or disorders.

**5.** An assay for screening for a modulator of neurodegenerative diseases, in particular Alzheimer's disease, or related diseases or disorders of one or more substances selected from the group consisting of

(i) a gene coding for a protein having SEQ ID NO.1, and/or
(ii) a transcription product of a gene coding for a protein having SEQ ID NO.1, and/or
(iii) a translation product of a gene coding for a protein having SEQ ID NO.1, and/or
(iv) a fragment, or derivative, or variant of (i) to (iii), wherein the variant has at least 95% sequence identity with the amino acid sequences of SEQ ID NO.1,

said method comprising:

(a) contacting a cell with a test compound;
(b) measuring the activity and/or level of one or more substances recited in (i) to (iv);
(c) measuring the activity and/or level of one or more substances recited in (i) to (iv) in a control cell not contacted with said test compound; and
(d) comparing the levels and/or activities of the substance in the cells of step (b) and (c), wherein an alteration in the activity and/or level of substances in the contacted cells indicates that the test compound is a modulator of said diseases or disorders.

**6.** A method of screening for a modulator of neurodegenerative diseases, in particular Alzheimer's disease, or related diseases or disorders of one or more substances selected from the group consisting of

(i) a gene coding for a protein having SEQ ID NO.1, and/or
(ii) a transcription product of a gene coding for a protein having SEQ ID NO.1, and/or
(iii) a translation product of a gene coding for a protein having SEQ ID NO.1, and/or
(iv) a fragment, or derivative, or variant of (i) to (iii), wherein the variant has at least 95% sequence identity with the amino acid sequences of SEQ ID NO.1,

said method comprising:

(a) administering a test compound to a non-human test animal which is predisposed to developing or has already developed symptoms of a neurodegenerative disease or related diseases or disorders in respect of the substances recited in (i) to (iv);

(b) measuring the activity and/or level of one or more substances recited in (i) to (iv);
(c) measuring the activity and/or level of one or more substances recited in (i) or (iv) in a matched non-human control animal which is predisposed to developing or has already developed symptoms of a neurodegenerative disease or related diseases or disorders in respect to the substances recited in (i) to (iv) and to which animal no such test compound has been administered;
(d) comparing the activity and/or level of the substance in the animals of step (b) and (c), wherein an alteration in the activity and/or level of substances in the test animal indicates that the test compound is a modulator of said diseases or disorders.

**7.** The method according to claim 6 wherein said test animal and/or said control animal is a recombinant animal which expresses a gene coding for a protein having SEQ ID NO.1, or a fragment, or a derivative, or a variant thereof, wherein the variant has at least 95% sequence identity with the amino acid sequences of SEQ ID NO.1 under the control of a transcriptional control element which is not the native gene transcriptional control element of a protein having SEQ ID NO.1.

**8.** An assay for testing a compound, preferably for screening a plurality of compounds to determine the degree of binding of said compounds to a translation product having SEQ ID NO.1, or to a fragment, or derivative, or variant thereof, wherein the variant has at least 95% sequence identity with the amino acid sequences of SEQ ID NO.1, said assay comprising the steps of:

(i) adding a liquid suspension of said translation product having SEQ ID NO.1, or a fragment, or derivative, or variant thereof, wherein the variant has at least 95% sequence identity with the amino acid sequences of SEQ ID NO.1 to a plurality of containers;
(ii) adding a detectable, in particular a fluorescently labelled compound or a plurality of detectable, in particular fluorescently labelled compounds to be screened for said binding to said plurality of containers;
(iii) incubating said translation product having SEQ ID NO.1, or said fragment, or derivative, or variant thereof, and said detectable, in particular fluorescently labelled compound or fluorescently labelled compounds;
(iv) measuring amounts of preferably fluorescence associated with said translation product having SEQ ID NO. 1, or with said fragment, or derivative, or variant thereof, wherein the variant has at least 95% sequence identity with the amino acid sequences of SEQ ID NO.1and
(v) determining the degree of binding by one or more of said compounds to said translation product having SEQ ID NO.1, or said fragment, or derivative, or variant thereof, wherein the variant has at least 95% sequence identity with the amino acid sequences of SEQ ID NO.1.

**9.** A protein molecule shown in SEQ ID NO. 1, or a fragment, or derivative, or variant thereof, wherein the variant has at least 95% sequence identity with the amino acid sequences of SEQ ID NO.1 for use as a diagnostic target for detecting a neurodegenerative disease, preferably Alzheimer's disease.

**10.** A protein molecule shown in SEQ ID NO.1, or a fragment, or derivative, or variant thereof, wherein the variant has at least 95% sequence identity with the amino acid sequences of SEQ ID NO.1 for use as a screening target for reagents or compounds preventing, or treating, or ameliorating a neurodegenerative disease, preferably Alzheimer's disease.

**11.** An antibody specifically immunoreactive with an immunogen, wherein said immunogen is a translation product of a gene coding for a protein having SEQ ID NO.1, or a fragment, or derivative, or variant thereof, wherein the variant has at least 95% sequence identity with the amino acid sequences of SEQ ID NO.1.

**12.** Use of an antibody of claim 11 for detecting a pathological state of a cell in a sample from a subject, comprising immunocytochemical staining of said cell with said antibody, wherein an altered degree of staining, or an altered staining pattern in said cell compared to a cell representing a known health status indicates a pathological state of said cell.

## Patentansprüche

**1.** Verfahren zum Diagnostizieren oder Prognostizieren einer neurodegenerativen Erkrankung bei einem Patienten oder zum Bestimmen, ob ein Patient ein erhöhtes Risiko hat, diese Erkrankung zu entwickeln, umfassend:

Bestimmen einer Konzentration und/oder einer Aktivität von:

(i) einem Transkriptionsprodukt eines Gens, das für ein Protein mit der SEQ ID Nr. 1 codiert; und/oder
(ii) einem Translationsprodukt eines Gens, das für ein Protein mit der SEQ ID Nr. 1 codiert; und/oder
(iii) einem Fragment oder Derivat oder einer Variante des Transcriptions- oder Translationsprodukts, wobei die Variante wenigstens 95% Sequenzidentität mit den Aminosäuresequenzen von SEQ ID Nr. 1 aufweist;

in einer Probe von dem Patienten und Vergleichen der Konzentration und/oder der Aktivität mit einem Referenzwert, der einen bekannten Krankheits- oder Gesundheitszustand darstellt, wodurch die neurodegenerative Erkrankung bei dem Patienten diagnostiziert oder prognostiziert wird oder bestimmt wird, ob der Patient ein erhöhtes Risiko hat, diese neurodegenerative Erkrankung zu entwickeln.

**2.** Verfahren gemäß Anspruch 1, wobei es sich bei der neurodegenerativen Erkrankung um die Alzheimer-Erkrankung handelt.

**3.** Kit zum Diagnostizieren oder Prognostizieren einer neurodegenerativen Erkrankung, insbesondere Alzheimer-Erkrankung, bei einem Patienten oder zum Bestimmen der Neigung oder Prädisposition eines Patienten, eine solche Krankheit zu entwickeln, wobei der Kit Folgendes umfasst:

a) wenigstens ein Reagens, das aus der Gruppe ausgewählt ist, die aus Folgendem besteht: (i) Reagentien, die selektiv ein Transkriptionsprodukt eines Gens, das für ein Protein mit der SEQ ID Nr. 1 codiert, nachweisen, und (ii) Reagentien, die selektiv ein Translationsprodukt eines Gens, das für ein Protein mit der SEQ ID Nr. 1 codiert, nachweisen; und
b) eine Anweisung zum Diagnostizieren oder Prognostizieren einer neurodegenerativen Erkrankung, insbesondere Alzheimer-Erkrankung, oder zum Bestimmen der Neigung oder Prädisposition eines Patienten, eine solche Erkrankung zu entwickeln, durch (i) Nachweisen der Konzentration oder der Aktivität oder sowohl der Konzentration als auch der Aktivität des Transkriptionsprodukts und/oder des Translationsprodukts eines Gens, das für ein Protein mit der SEQ ID Nr. 1 codiert, in einer Probe von dem Patienten; und (ii) Diagnostizieren oder Prognostizieren einer neurodegenerativen Erkrankung, insbesondere Alzheimer-Erkrankung, oder Bestimmen der Neigung oder Prädisposition des Patienten, eine solche Erkrankung zu entwickeln, wobei eine variierte Konzentration oder Aktivität oder sowohl Konzentration als auch Aktivität des Transkriptionsprodukts und/oder des Translationsprodukts im Vergleich zu einem Referenzwert, der einen bekannten Gesundheitszustand darstellt, oder eine Konzentration oder Aktivität oder sowohl Konzentration als auch Aktivität des Transkriptionsprodukts und/oder Translationsprodukts, die einem Referenzwert, der einen bekannten Krankheitszustand darstellt, ähnlich oder gleich sind, eine Diagnose oder Prognose einer neurodegenerativen Erkrankung, insbesondere Alzheimer-Erkrankung, oder eine erhöhte Neigung oder Prädisposition, eine solche Krankheit zu entwickeln, anzeigt.

**4.** Rekombinantes nichthumanes Tier, das eine nichtnative Gensequenz, die für ein Protein mit der SEQ ID Nr. 1 codiert, oder ein Fragment oder Derivat oder eine Variante davon umfasst, wobei die Variante wenigstens 95% Sequenzidentität mit den Aminosäuresequenzen von SEQ ID Nr. 1 aufweist, wobei das Tier erhältlich ist durch:

(i) Bereitstellen eines Genzielsteuerungskonstrukts, das die Gensequenz und eine Selektionsmarkersequenz umfasst; und
(ii) Einführen des Zielsteuerungskonstrukts in eine Stammzelle eines nichthumanen Tiers; und
(iii) Einführen der Stammzelle des nichthumanen Tiers in einen nichthumanen Embryo; und
(iv) Transplantieren des Embryos in ein scheinträchtiges nichthumanes Tier; und
(v) Entwickelnlassen des Embryos bis zur Reife; und
(vi) Identifizieren eines genetisch veränderten nichthumanen Tiers, dessen Genom eine Modifikation der Gensequenz in beiden Allelen umfasst; und
(vii) Aufziehen des genetisch veränderten nichthumanen Tiers von Schritt (vi), so dass man ein genetisch verändertes nichthumanes Tier erhält, dessen Genom eine Modifikation des endogenen Gens umfasst, wobei der Eingriff dazu führt, dass das nichthumane Tier eine Prädisposition aufweist, eine neurodegenerative Erkrankung oder verwandte Erkrankungen oder Störungen zu entwickeln.

**5.** Assay zum Suchen nach einem Modulator von neurodegenerativen Krankheiten, insbesondere Alzheimer-Erkrankung, oder verwandten Erkrankungen oder Störungen durch Screening von einer oder mehreren Substanzen, die aus der Gruppe ausgewählt sind, die aus Folgenden besteht:

(i) einem Gen, das für ein Protein mit der SEQ ID Nr. 1 codiert; und/oder
(ii) einem Transkriptionsprodukt eines Gens, das für ein Protein mit der SEQ ID Nr. 1 codiert; und/oder
(iii) einem Translationsprodukt eines Gens, das für ein Protein mit der SEQ ID Nr. 1 codiert; und/oder
(iv) einem Fragment oder Derivat oder einer Variante von (i) bis (iii), wobei die Variante wenigstens 95% Sequenzidentität mit den Aminosäuresequenzen von SEQ ID Nr. 1 aufweist;

wobei das Verfahren Folgendes umfasst:

a) In-Kontakt-Bringen einer Zelle mit einer Testverbindung;
b) Messen der Aktivität und/oder der Konzentration von einer oder mehreren Substanzen, die in (i) bis (iv) genannt sind;
c) Messen der Aktivität und/oder der Konzentration von einer oder mehreren Substanzen, die in (i) bis (iv) genannt sind, in einer Kontrollzelle, die nicht mit der Testverbindung in Kontakt gebracht wurde; und
d) Vergleichen der Konzentrationen und/oder Aktivitäten der Substanz in den Zellen der Schritte (b) und (c), wobei eine Änderung der Aktivität und/oder der Konzentration der Substanzen in den kontaktierten Zellen anzeigt, dass die Testverbindung ein Modulator der Erkrankungen oder Störungen ist.

**6.** Verfahren zum Suchen nach einem Modulator von neurodegenerativen Erkrankungen, insbesondere Alzheimer-Erkrankungen, oder verwandten Erkrankungen oder Störungen durch Screening von einer oder mehreren Substanzen, die aus der Gruppe ausgewählt sind, die aus Folgenden besteht:

(i) einem Gen, das für ein Protein mit der SEQ ID Nr. 1 codiert; und/oder
(ii) einem Transkriptionsprodukt eines Gens, das für ein Protein mit der SEQ ID Nr. 1 codiert; und/oder
(iii) einem Translationsprodukt eines Gens, das für ein Protein mit der SEQ ID Nr. 1 codiert; und/oder
(iv) einem Fragment oder Derivat oder einer Variante von (i) bis (iii), wobei die Variante wenigstens 95% Sequenzidentität mit den Aminosäuresequenzen von SEQ ID Nr. 1 aufweist;

wobei das Verfahren Folgendes umfasst:

a) das Verabreichen einer Testverbindung an ein nichthumanes Testtier, das prädisponiert ist, eine neurodegenerative Erkrankung oder verwandte Erkrankungen oder Störungen zu entwickeln, oder bereits entsprechende Symptome entwickelt hat, in Bezug auf die Substanzen, die in (i) bis (iv) genannt sind;
b) das Messen der Aktivität und/oder der Konzentration von einer oder mehreren Substanzen, die in (i) bis (iv) genannt sind;
c) das Messen der Aktivität und/oder der Konzentration von einer oder mehreren Substanzen, die in (i) oder (iv) genannt sind, in einem angepassten nichthumanen Kontrolltier, das prädisponiert ist, Symptome einer neurodegenerativen Krankheit oder von verwandten Krankheiten oder Störungen zu entwickeln, oder sie bereits entwickelt hat, in Bezug auf die Substanzen, die in (i) bis (iv) genannt sind, wobei dem Kontrolltier keine solche Testverbindung verabreicht wurde;
d) das Vergleichen der Aktivität und/oder der Konzentration der Substanz in den Tieren der Schritte (b) und (c), wobei eine Änderung der Aktivität und/oder der Konzentration der Substanzen bei dem Testtier anzeigt, dass die Testverbindung ein Modulator der Erkrankungen oder Störungen ist.

**7.** Verfahren gemäß Anspruch 6, wobei das Testtier und/oder das Kontrolltier ein rekombinantes Tier, das ein Gen, das für ein Protein mit der SEQ ID Nr. 1 codiert, oder ein Fragment oder ein Derivat oder eine Variante davon, wobei die Variante wenigstens 95% Sequenzidentität mit den Aminosäuresequenzen von SEQ ID Nr. 1 aufweist, unter der Kontrolle eines transkriptionsregulatorischen Elements, bei dem es sich nicht um das native transkriptionsregulatorische Element eines Proteins mit der SEQ ID Nr. 1 handelt, exprimiert.

**8.** Assay zum Testen einer Verbindung, vorzugsweise zum Durchmustern (Screening) einer Menge von Verbindungen, zum Bestimmen des Grades der Bindung der Verbindungen an ein Translationsprodukt mit SEQ ID Nr. 1 oder an ein Fragment oder Derivat oder eine Variante davon, wobei die Variante wenigstens 95% Sequenzidentität mit den Aminosäuresequenzen von SEQ ID Nr. 1 aufweist, wobei der Assay die folgenden Schritte umfasst:

(i) Hinzufügen einer flüssigen Suspension des Translationsprodukts mit der SEQ ID Nr. 1 oder des Fragments oder Derivats oder der Variante davon, wobei die Variante wenigstens 95% Sequenzidentität mit den Aminosäuresequenzen von SEQ ID Nr. 1 aufweist, zu einer Menge von Behältern;
(ii) Hinzufügen einer nachweisbaren, insbesondere fluoreszenzmarkierten Verbindung oder einer Menge von nachweisbaren, insbesondere fluoreszenzmarkierten Verbindungen, die in Bezug auf die Bindung an die Menge von Behältern durchmustert werden sollen;

(iii) Inkubieren des Translationsprodukts mit der SEQ ID Nr. 1 oder des Fragments oder Derivats oder der Variante davon und der nachweisbaren, insbesondere fluoreszenzmarkierten Verbindung oder der fluoreszenzmarkierten Verbindungen;

(iv) Messen der Mengen an vorzugsweise Fluoreszenz, die mit dem Translationsprodukt mit der SEQ ID Nr. 1 oder dem Fragment oder Derivat oder der Variante davon, wobei die Variante wenigstens 95% Sequenzidentität mit den Aminosäuresequenzen von SEQ ID Nr. 1 aufweist, verbunden ist; und

(v) Bestimmen des Grades der Bindung durch eine oder mehrere der Verbindungen an das Translationsprodukt mit SEQ ID Nr. 1 oder das Fragment oder Derivat oder die Variante davon, wobei die Variante wenigstens 95% Sequenzidentität mit den Aminosäuresequenzen von SEQ ID Nr. 1 aufweist.

**9.** Proteinmolekül, das in SEQ ID Nr. 1 gezeigt ist, oder ein Fragment oder Derivat oder eine Variante davon, wobei die Variante wenigstens 95% Sequenzidentität mit den Aminosäuresequenzen von SEQ ID Nr. 1 aufweist, zur Verwendung als diagnostisches Target zum Nachweisen einer neurodegenerativen Erkrankung, vorzugsweise Alzheimer-Erkrankung.

**10.** Proteinmolekül, das in SEQ ID Nr. 1 gezeigt ist, oder ein Fragment oder Derivat oder eine Variante davon, wobei die Variante wenigstens 95% Sequenzidentität mit den Aminosäuresequenzen von SEQ ID Nr. 1 aufweist, zur Verwendung als Screening-Target für Reagentien oder Verbindungen, die eine neurodegenerative Erkrankung, vorzugsweise Alzheimer-Erkrankung, verhindern oder behandeln oder lindern.

**11.** Antikörper, der gegenüber einem Immunogen spezifisch immunreaktiv ist,
wobei das Immunogen ein Translationsprodukt eines Gens, das für ein Protein mit der SEQ ID Nr. 1 codiert, oder ein Fragments oder Derivat oder eine Variante davon ist, wobei die Variante wenigstens 95% Sequenzidentität mit den Aminosäuresequenzen von SEQ ID Nr. 1 aufweist.

**12.** Verwendung eines Antikörpers gemäß Anspruch 11 zum Nachweis eines pathologischen Zustands einer Zelle in einer Probe von einem Patienten, der das immunozytochemische Anfärben der Zelle mit dem Antikörper umfasst, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt.

**Revendications**

**1.** Procédé pour diagnostiquer ou pronostiquer une maladie neurodégénérative chez un sujet, ou pour déterminer si un sujet présente un risque accru de développer ladite maladie, comprenant :

la détermination d'une concentration et/ou d'une activité

(i) d'un produit de transcription d'un gène codant une protéine de séquence SEQ ID N° 1, et/ou
(ii) d'un produit de traduction d'un gène codant une protéine de séquence SEQ ID N° 1, et/ou
(iii) d'un fragment, d'un dérivé ou d'une variante dudit produit de transcription ou de traduction, dans lequel la variante présente une identité de séquence d'au moins 95% avec les séquences d'acides aminés de SEQ ID N° 1

dans un échantillon prélevé sur ledit sujet et la comparaison de ladite concentration et/ou de ladite activité à une valeur de référence représentant une maladie ou un état de santé connu, pour diagnostiquer ou pronostiquer ainsi ladite maladie neurodégénérative chez ledit sujet, ou pour déterminer si ledit sujet présente un risque accru de développer ladite maladie neurodégénérative.

**2.** Procédé selon la revendication 1, dans lequel ladite maladie neurodégénérative est la maladie d'Alzheimer.

**3.** Trousse pour diagnostiquer ou pronostiquer une maladie neurodégénérative, en particulier la maladie d'Alzheimer, chez un sujet, ou pour déterminer la propension ou la prédisposition d'un sujet à développer une telle maladie, ladite trousse comprenant :

(a) au moins un réactif qui est choisi dans le groupe constitué par (i) les réactifs qui détectent de manière sélective un produit de transcription d'un gène codant une protéine de séquence SEQ ID N° 1 et (ii) les réactifs qui détectent de manière sélective un produit de traduction d'un gène codant une protéine de séquence SEQ ID N° 1 ; et

(b) des instructions pour le diagnostic ou le pronostic d'une maladie neurodégénérative, en particulier la maladie d'Alzheimer, ou la détermination de la propension ou de la prédisposition d'un sujet à développer une telle maladie (i) en détectant une concentration, une activité ou à la fois ladite concentration et ladite activité dudit produit de transcription et/ou dudit produit de traduction d'un gène codant une protéine de séquence SEQ ID N°1, dans un échantillon prélevé sur ledit sujet ; et (ii) en diagnostiquant ou en pronostiquant une maladie neurodégénérative, en particulier la maladie d'Alzheimer, ou en déterminant la propension ou la prédisposition dudit sujet à développer une telle maladie, dans lequel une concentration variable, une activité ou à la fois ladite concentration et ladite activité dudit produit de transcription et/ou dudit produit de traduction est différente d'une valeur de référence représentant un état de santé connu ; ou une concentration, une activité ou à la fois ladite concentration et ladite activité dudit produit de transcription et/ou dudit produit de traduction est similaire ou égale à une valeur de référence représentant un état pathologique connu indique un diagnostic ou un pronostic d'une maladie neurodégénérative, en particulier la maladie d'Alzheimer, ou une plus grande propension ou prédisposition à développer une telle maladie.

**4.** Animal non humain obtenu par recombinaison comprenant une séquence de gènes non native codant une protéine de séquence SEQ ID N° 1, ou un fragment, un dérivé ou une variante de celle-ci, dans lequel la variante présente une identité de séquence d'au moins 95% avec les séquences d'acides aminés de SEQ ID N° 1, ledit animal pouvant être obtenu :

(i) en fournissant un système artificiel de ciblage du gène comprenant ladite séquence de gènes et une séquence marqueur sélectionnable, et
(ii) en introduisant ledit système artificiel de ciblage dans une cellule souche d'un animal non humain, et
(iii) en introduisant ladite cellule souche d'animal non humain dans un embryon non humain, et
(iv) en implantant ledit embryon dans un animal non humain en pseudogestation, et
(v) en laissant ledit embryon se développer jusqu'à terme, et
(vi) en identifiant un animal non humain génétiquement modifié dont le génome comprend une variante de ladite séquence de gènes dans les deux allèles, et
(vii) en élevant l'animal non humain génétiquement modifié de l'étape (vi) pour obtenir un animal non humain génétiquement modifié dont le génome comprend une modification dudit gène endogène, dans lequel ladite perturbation entraîne chez ledit animal non humain une prédisposition à développer des symptômes d'une maladie neurodégénérative ou de troubles ou maladies apparentés.

**5.** Dosage de criblage d'un modulateur de maladies neurodégénératives, en particulier la maladie d'Alzheimer ou de troubles ou maladies apparentés, d'une ou plusieurs substances choisies dans le groupe constitué par

(i) un gène codant une protéine de séquence SEQ ID N° 1, et/ou
(ii) d'un produit de transcription d'un gène codant une protéine de séquence SEQ ID N° 1, et/ou
(iii) d'un produit de traduction d'un gène codant une protéine de séquence SEQ ID N° 1, et/ou
(iv) un fragment, un dérivé ou une variante de (i) à (iii), dans lequel la variante présente une identité de séquence d'au moins 95% avec les séquences d'acides aminés de SEQ ID N° 1,

ledit procédé comprenant les étapes consistant à :

(a) mettre en contact une cellule avec un composé d'essai ;
(b) mesurer l'activité et/ou la concentration d'une ou plusieurs des substances mentionnées en (i) à (iv) ;
(c) mesurer l'activité et/ou la concentration d'une ou plusieurs des substances mentionnées en (i) à (iv) dans une cellule témoin n'ayant pas été mise en contact avec ledit composé d'essai ; et
(d) comparer la concentration et/ou l'activité de la substance dans les cellules des étapes (b) et (c), dans lequel une modification de l'activité et/ou de la concentration des substances dans les cellules mises en contact indique que le composé d'essai est un modulateur desdits troubles ou maladies.

**6.** Procédé de criblage d'un modulateur de maladies neurodégénératives, en particulier la maladie d'Alzheimer ou de troubles ou maladies apparentés, d'une ou plusieurs substances choisies dans le groupe constitué par

(i) un gène codant une protéine de séquence SEQ ID N° 1, et/ou

(ii) d'un produit de transcription d'un gène codant une protéine de séquence SEQ ID N° 1, et/ou
(iii) d'un produit de traduction d'un gène codant une protéine de séquence SEQ ID N° 1, et/ou
(iv) un fragment, un dérivé ou une variante de (i) à (iii), dans lequel la variante présente une identité de séquence d'au moins 95% avec les séquences d'acides aminés de SEQ ID N° 1,
ledit procédé comprenant les étapes consistant à :
(a) administrer un composé d'essai à un animal d'essai non humain qui est prédisposé à développer ou qui a déjà développé des symptômes d'une maladie neurodégénérative ou de troubles ou maladies apparentés en ce qui concerne les substances mentionnées en (i) à (iv) ;
(b) mesurer l'activité et/ou la concentration d'une ou plusieurs des substances mentionnées en (i) à (iv) ;
(c) mesurer l'activité et/ou la concentration d'une ou plusieurs des substances mentionnées en (i) ou (iv) chez un animal d'essai non humain correspondant qui est prédisposé à développer ou qui a déjà développé des symptômes d'une maladie neurodégénérative ou de troubles ou maladies apparentés en ce qui concerne les substances mentionnées en (i) à (iv), aucun composé d'essai n'ayant été administré à cet animal ;
(d) comparer l'activité et/ou la concentration de la substance chez les animaux des étapes (b) et (c), dans lequel une modification de l'activité et/ou de la concentration des substances chez l'animal d'essai indique que le composé d'essai est un modulateur desdits troubles ou maladies.

**7.** Procédé selon la revendication 6, dans lequel ledit animal d'essai et/ou ledit animal témoin est un animal obtenu par recombinaison qui exprime un gène codant une protéine de séquence SEQ ID N° 1, ou un fragment, un dérivé ou une variante de celui-ci, dans lequel la variante présente une identité de séquence d'au moins 95% avec les séquences d'acides aminés de SEQ ID N° 1 sous la régulation d'un élément de régulation transcriptionnelle qui n'est pas l'élément de régulation transcriptionnelle du gène natif d'une protéine de séquence SEQ ID N° 1.

**8.** Dosage pour l'évaluation d'un composé, de préférence pour le criblage d'une pluralité de composés afin de déterminer le degré de liaison desdits composés à un produit de traduction de séquence SEQ ID N° 1, ou à un fragment, un dérivé ou une variante de celui-ci, dans lequel la variante présente une identité de séquence d'au moins 95% avec les séquences d'acides aminés de SEQ ID N° 1, ledit dosage comprenant les étapes consistant à :

(i) ajouter une suspension liquide dudit produit de traduction de séquence SEQ ID N° 1, ou un fragment, un dérivé ou une variante de celui-ci, dans lequel la variante présente une identité de séquence d'au moins 95% avec les séquences d'acides aminés de SEQ ID N° 1, à une pluralité de récipients ;
(ii) ajouter un composé détectable, en particulier un composé marqué par fluorescence ou une pluralité de composés détectables, en particulier de composés marqués par fluorescence devant être criblés pour évaluer ladite liaison à ladite pluralité de récipients ;
(iii) incuber ledit produit de traduction de séquence SEQ ID N° 1, ou ledit fragment, dérivé ou variante de celui-ci, et ledit marqueur détectable, en particulier un composé marqué par fluorescence ou des composés marqués par fluorescence ;
(iv) mesurer des niveaux de fluorescence de préférence associée audit produit de traduction de séquence SEQ ID N° 1, ou audit fragment, dérivé ou variante de celui-ci, dans lequel la variante présente une identité de séquence d'au moins 95% avec les séquences d'acides aminés de SEQ ID N° 1 et
(v) déterminer le degré de liaison d'un ou plusieurs desdits composés audit produit de traduction de séquence SEQ ID N° 1, ou audit fragment, dérivé ou variante de celui-ci, dans lequel la variante présente une identité de séquence d'au moins 95% avec les séquences d'acides aminés de SEQ ID N° 1.

**9.** Molécule protéique illustrée par SEQ ID N° 1, ou un fragment, un dérivé ou une variante de celle-ci, dans laquelle la variante présente une identité de séquence d'au moins 95% avec les séquences d'acides aminés de SEQ ID N° 1, pour une utilisation en tant que cible diagnostique pour dépister une maladie neurodégénérative, de préférence la maladie d'Alzheimer.

**10.** Molécule protéique illustrée par SEQ ID N° 1, ou un fragment, un dérivé ou une variante de celle-ci, dans laquelle la variante présente une identité de séquence d'au moins 95% avec les séquences d'acides aminés de SEQ ID N° 1, pour une utilisation en tant que cible de criblage pour des réactifs ou des composés prévenant, traitant ou améliorant une maladie neurodégénérative, de préférence la maladie d'Alzheimer.

**11.** Anticorps spécifiquement immunoréactif avec un agent immunogène, dans lequel ledit agent immunogène est un produit de traduction d'un gène codant une protéine de séquence SEQ ID N° 1, ou un fragment, un dérivé ou une variante de celle-ci, dans lequel la variante présente une identité de séquence d'au moins 95% avec les séquences d'acides aminés de SEQ ID N° 1.

**12.** Utilisation de l'anticorps selon la revendication 11 pour dépister un état pathologique d'une cellule dans un échantillon prélevé sur un sujet, comprenant la coloration immunocytochimique de ladite cellule avec ledit anticorps, dans lequel un degré de coloration modifié ou un schéma de coloration modifié dans ladite cellule par rapport à une cellule représentant un état de santé connu indique un état pathologique de ladite cellule.

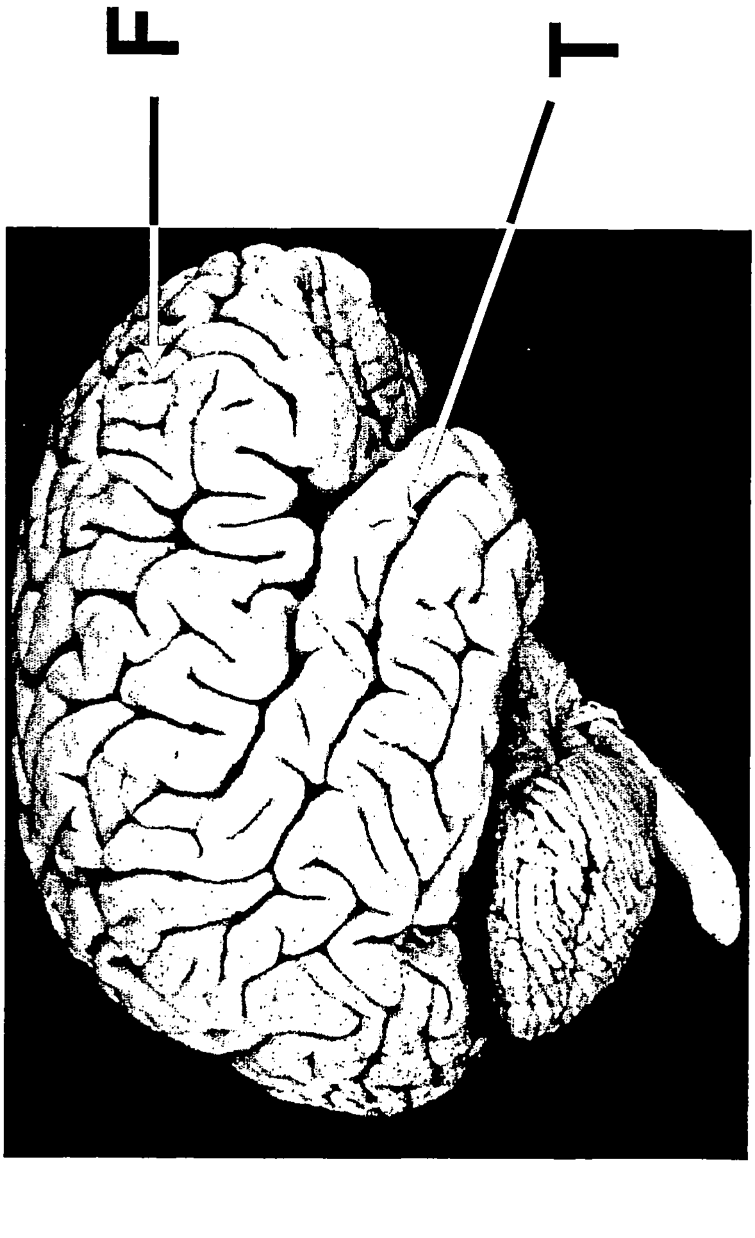


Fig. 1: Identification of Genes Involved in Alzheimer's Disease Pathology

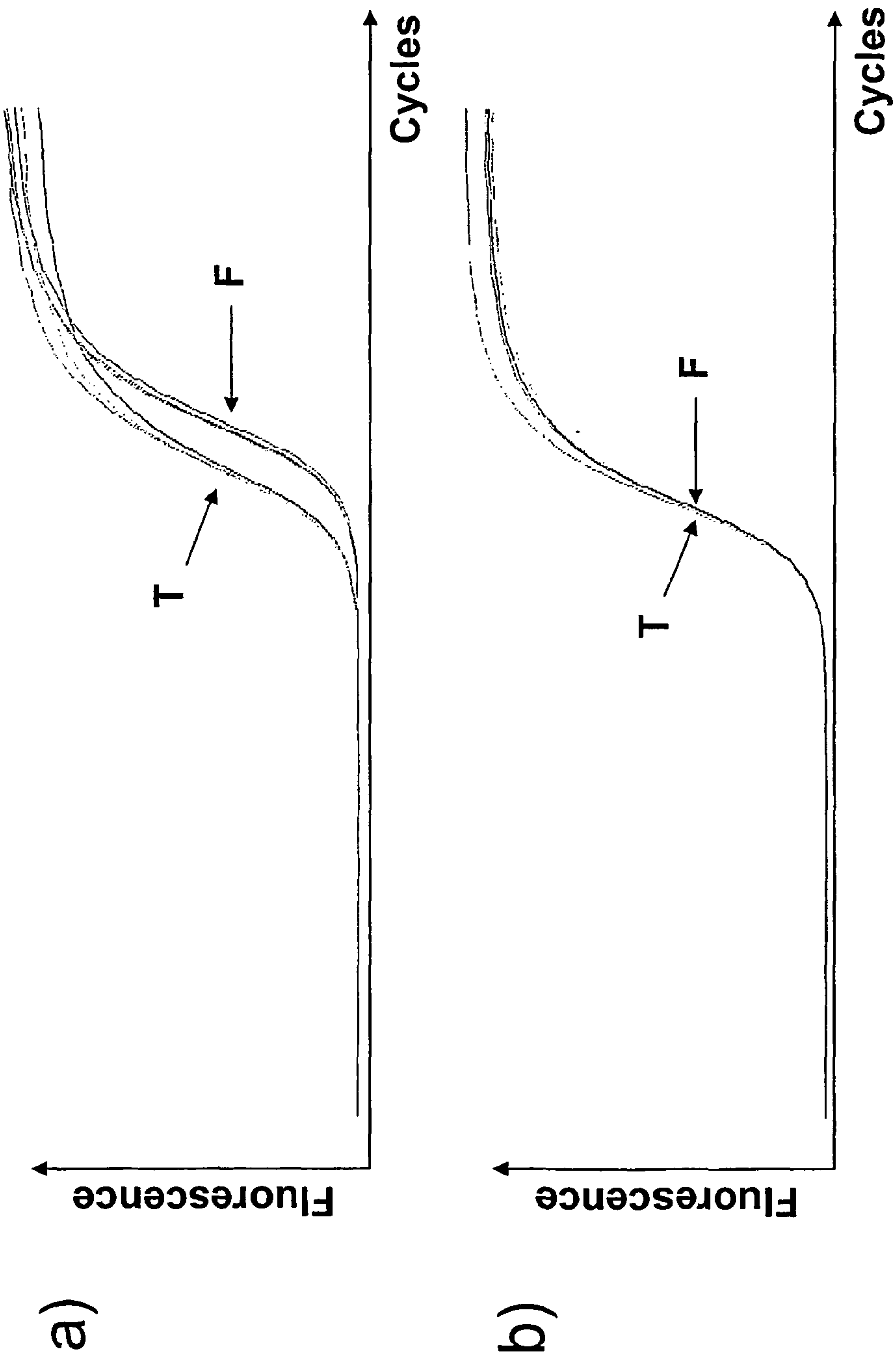


**Fig. 2 Verification of differential expression of Ensadin-0581 by quantitative RT-PCR**

**Fig. 3 Verification of differential expression of Ensadin-0581 by quantitative RT-PCR**

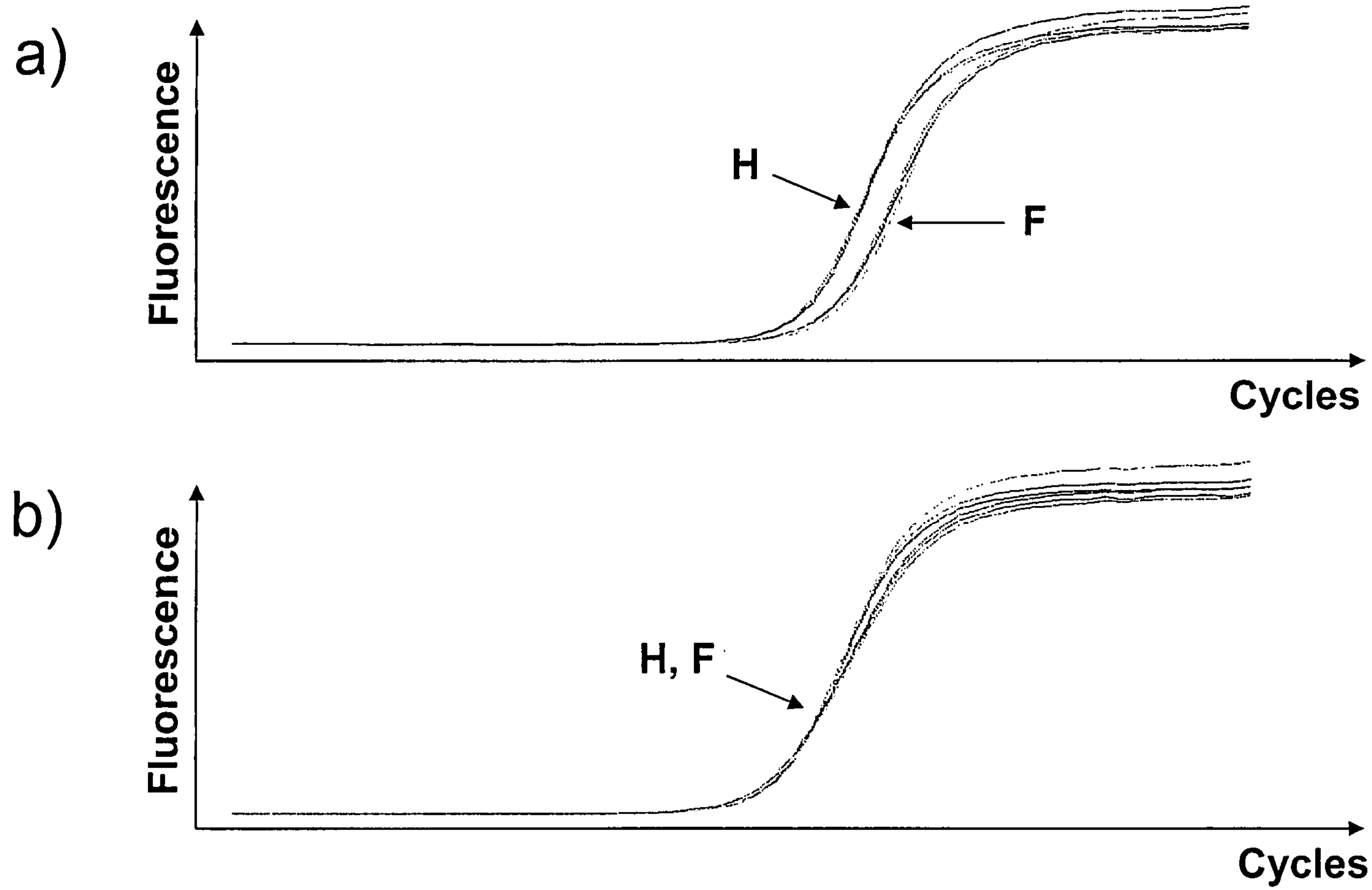

# Fig. 4 SEQ ID NO. 1; amino acid sequence of human Ensadin-0581 protein

**Length: 620 aa**

```
  1 MPSERCLSIQ EMLTGQRLCH SESHNDSVLA ALNQQRSDGI LCDITLIAEE
 51 QKFHAHKAVL AACSDYFRAM FSLCMVESGA DEVNLHGVTS LGLKQALEFA
101 YTGQILLEPG VIQDVLAAGS HLQLLELLNL CSHYLIQELN SFNYLDLYRL
151 ADLFNLTLLE KAVIDFLVKH LSELLKSRPE EVLTLPYCLL QEVLKSDRLT
201 SLSEEQIWQL AVRWLEHNCH YQYMDELLQY IRFGLMDVDT LHTVALSHPL
251 VQASETATAL VNEALEYHQS IYAQPVWQTR RTKPRFQSDT LYIIGGKKRE
301 VCKVKELRYF NPVDQENALI AAIANWSELA SMPVGRSHHC VAVMGDFLFV
351 AGGEVEHASG RTCAVRTACR YDPRSNSWAE IAPMKNCREH FVLGAMEEYL
401 YAVGGRNELR QVLPTVERYC PKKNKWTFVQ SFDRSLSCHA GYVADGLLWI
451 SGGVTNTAQY QNRLMVYEPN QNKWISRSPM LQRRVYHSMA AVQRKLYVLG
501 GNDLDYNNDR ILVRHIDSYN IDTDQWTRCN FNLLTGQNES GVAVHNGRIY
551 LVGGYSIWTN EPLACIQVLD VSREGKEEVF YGPTLPFASN GIAACFLPAP
601 YFTCPNLQTL QVPHHRIGTI
```

# Fig. 5 SEQ ID NO. 2; nucleotide sequence of human Ensadin-0581 cDNA

**Length: 3693 bp**

```
   1  ATTTTTACTA GGGAGCAGTT TCCCCGCGCG ACAGTTCGGG AGCGCGCAGG
  51  CAGTCGCGCG CACACACGCA CGCAGGCACA CACACACACA CACACACACA
 101  CACTTTCGCA CACACAAACA CGCTAGGACG CTCGTCTTCG CTACTGCATC
 151  CCCGAACCAG CAGAGCGAAG CTACTGCGGG TTCTGTTAAC CTCAGCATCG
 201  TGGGGCGAAG CAGAGCCATT GTGCATCAAG GAGAGGCCGG TGCCTGCGCT
 251  GCCGTCTCTG GCACCTAACC CAGCAGACCG CTCACCCCAT CGGCTGGAAT
 301  GCTTGCTGAT TCCTCTGCAC ACTTCTAAGG CTGAGAACCT GAGGAACCCA
 351  GCTGGAAAAT GCCGTCTGAA CGCTGCCTCA GTATTCAAGA AATGCTGACA
 401  GGCCAGAGGC TCTGCCACTC CGAATCTCAC AATGACAGTG TCCTGGCAGC
 451  GCTGAATCAG CAGAGGAGTG ATGGCATCCT CTGCGACATC ACCCTGATTG
 501  CTGAGGAACA GAAATTCCAT GCTCACAAGG CAGTCCTAGC AGCATGCAGT
 551  GACTATTTCC GGGCAATGTT CAGTCTTTGT ATGGTGGAAA GTGGAGCTGA
 601  TGAGGTTAAT TTGCACGGTG TGACCAGCCT TGGCTTAAAG CAGGCTCTGG
 651  AGTTTGCATA CACAGGACAG ATTTTGCTGG AGCCAGGTGT GATCCAGGAT
 701  GTGCTAGCAG CGGGCAGTCA CCTACAGCTG TTGGAGCTTC TCAATTTATG
 751  CTCCCACTAT CTCATCCAGG AATTAAATAG CTTTAATTAC TTGGATCTGT
 801  ACAGACTTGC TGACCTCTTT AACCTCACTT TGTTGGAGAA GGCAGTGATC
 851  GATTTCTTAG TGAAACATCT CTCTGAACTC CTGAAGAGCC GCCCAGAAGA
 901  AGTTCTAACG CTTCCCTATT GCCTGCTTCA GGAGGTGCTG AAGAGCGACC
 951  GCCTGACCTC CCTGAGTGAA GAGCAGATCT GGCAGCTAGC TGTGAGGTGG
1001  TTGGAACACA ACTGCCACTA CCAGTACATG GACGAGCTCC TGCAATACAT
1051  CCGCTTTGGC CTAATGGATG TGGATACTCT CCATACAGTT GCCCTGTCCC
1101  ACCCCCTTGT CCAAGCAAGT GAGACTGCAA CAGCCCTTGT CAACGAGGCC
1151  CTGGAATACC ACCAGAGCAT CTATGCACAG CCTGTCTGGC AGACTCGCAG
1201  GACCAAACCA CGATTCCAGT CAGACACTCT GTATATCATT GGTGGGAAAA
1251  AGCGCGAGGT CTGCAAGGTC AAGGAACTTC GGTACTTCAA TCCTGTTGAT
1301  CAGGAGAATG CTCTCATAGC TGCCATTGCC AACTGGAGTG AGCTGGCTCC
1351  CATGCCTGTG GGAAGGAGCC ACCATTGTGT GGCAGTCATG GGGGACTTCC
1401  TGTTTGTGGC AGGAGGGGAA GTTGAGCATG CCAGTGGCCG GACGTGTGCT
1451  GTGAGGACTG CCTGTCGCTA TGACCCCCGC AGTAATTCCT GGGCAGAGAT
1501  AGCACCCATG AAAAACTGCC GGGAGCATTT TGTGCTGGGT GCCATGGAGG
1551  AATACCTCTA TGCAGTTGGG GGCAGAAATG AACTGCGCCA GGTTCTGCCT
1601  ACAGTTGAGC GATATTGCCC CAAGAAGAAC AAATGGACTT TTGTTCAGTC
1651  CTTTGACAGA TCCCTTTCAT GCCATGCTGG ATATGTGGCT GATGGTCTTC
1701  TTTGGATATC AGGTGGAGTA ACTAATACGG CACAATATCA GAACAGGCTA
1751  ATGGTGTATG AACCTAACCA GAATAAGTGG ATAAGCCGTA GCCCCATGCT
1801  GCAGAGAAGG GTCTACCATT CCATGGCTGC TGTACAAAGG AAGCTTTATG
1851  TTCTTGGAGG CAATGACCTA GACTACAATA ATGACCGGAT CCTTGTGCGC
```

```
1901  CATATAGATT CTTACAACAT AGACACTGAC CAGTGGACAC GTTGTAATTT
1951  CAACCTGCTG ACTGGCCAAA ATGAATCTGG AGTTGCTGTC CATAATGGGA
2001  GAATATATTT AGTTGGTGGA TATTCAATTT GGACAAATGA GCCTCTGGCT
2051  TGTATCCAGG TACTGGATGT AAGCAGAGAA GGCAAAGAAG AAGTATTCTA
2101  TGGGCCTACA CTCCCTTTTG CTTCCAATGG AATAGCAGCA TGCTTCCTTC
2151  CAGCTCCATA TTTTACATGC CCTAACCTTC AAACTCTTCA AGTGCCTCAT
2201  CACAGGATTG GCACCATCTG AAAAGCCAAG CCATCATGAA CAGGAGGAAA
2251  ACATAGCTCT GACTGTTGGA TACTGGGCAT GAAAAGACTC AGTGCTCCAT
2301  GCTTCCTTGT CTTGCTTTAT AGGTCTTATA TTCGGATAAA TTTAAGCAAA
2351  AAATGAACAA TTTTCTAAAA TACGTTATTG AAAACTCGTC ACCCTTCTCA
2401  GTGTATGTCA ACATTCAATA TGTATGACTT TTATTGTGGT ATAGCTTAGT
2451  GCCAATTTAA GGTATATTGT GTTCCATGGG TCTTTAGAGC ATTCTTTGTA
2501  CACTTTTTCT AATCAGCACT GTCTTAAGAC AACATAACAC TGTTAGTAAG
2551  GCAATTTATT GGACACAATG GCATCGATGA TCTTAAAAAT ATATATATTC
2601  TGTACTTAAT CCCTTTATTA TTTAAAGCCG GGCTTGTAAT TTTTCTTTTT
2651  AAAATTTAAC ATTATGCAGC CCTGCTCAGT AGAGACTCAC TCAGCATTAT
2701  GACATCATAA ATTTTTAAAT GCCATATTTT ATTCAAGGTG ATACGAATAA
2751  TAATAGACAT ACACTGGAAC AGCAGTTGTT GAAATACCCA GATTTTTCAC
2801  CTTGGTTACT CTGACCTTGG GTCAGAACCT AACTTTACAG TGCTTCAGTT
2851  TTCCCACCTT TTAAGGAGGA TAATTACTGA TTTCCACGTT AATTTAAAAG
2901  AACATTATGA GGATTAGGCG AACACTTTGT AATACTTTGC CCAAGAAAAA
2951  GAAAAATGTT GTTACTGAAA TTATAAGGTA TTTTATTGTC TTTAGAACCT
3001  TGTAATTGAA CAGGGAGATT ATTTTTGAAG TTTAAAAATT ACTAGATAGA
3051  ATTTGGAAAC ATGTTGTGGA ATAAATGATT CTAAAAAGCA TGTGAAAGAG
3101  ACACACAATG AGGAAATAGG GAGTTTCTCA AAATCTAAAC TTGTACAGGT
3151  ATCTAAACTT GCCTCAAGTA ACTCTCAGAT GTCTTATTTA AAATCATTTA
3201  GTGAAAAATG ACATTTAAAG CTAAAAAATA ATTTAGTTGT ACTAATTATG
3251  GTACCATAAA GTGCTTTGAC ATAAATTTGA ACCTAGAATT GGCAGTTCTA
3301  ATAAAAGTTT TTCCACTTTG TTAAAGGTTA TGTCAATCTT GAGTGCTTGT
3351  GGAATTCTTC ACCACCAATA CATATTTTAT TACTATCTCT TTTAATAATG
3401  CATAGGAATT CTTTTTTAGA CTAGTTTTTG GGTTTGCTTT GTCTATAACA
3451  AAAAATAAAT ACAACAACTT AATAATCACA TTTGAAAACA AATTTAACAA
3501  GTATAATGTG AGTTTTTCTT TTTTCCTAAC TTCCTCAGGA CCTAGGTCAC
3551  CTTTATTAAA AGGAAGAATT CACTCTTTTT TCCTTGGCAG TTTAATCATT
3601  CAGGAATCCC TCTCTTAAAG GAAATTGTTA TTTTATTCAT GTAACCTTTT
3651  TTTGTAATCA AAAGTGAATA AAAACGATCT TTTTGTCTTA CAG
```

## Fig. 6 SEQ ID NO. 3

**Length: 328 bp**

```
  1   CCAATACATA TTTTATTACT ATCTCTTTTA ATAATGCATA GGAATTCTTT
 51   TTTAGACTAG TTTTTGGGTT TGCTTTGTCT ATAACAAAAA ATAAATACAA
101   CAACTTAATA ATCACATTTG AAAACAAATT TAACAAGTAT AATGTGAGTT
151   TTTCTTTTTT CCTAACTTCC TCAGGACTTA GGTCACCTTT ATTAAAAGGA
201   AGAATTCACT CTTTTTTCCT TGGCAGTTTA ATCATTCAGG AATCCCTCTC
251   TTAAAGGAAA TTGTTATTTT ATTCATGTAA CCTTTTTTTG TAATCAAAAG
301   TGAATAAAAA CGATCTTTTT GTCTTACA
```

# Fig. 7 Alignment of SEQ ID NO. 3 with human Ensadin- 0581 cDNA (consensus sequence GenBank accession numbers AK055292 and AB067487)

**Length: 328 bp**

```
                .         .         .         .         .
   1 CCAATACATATTTTATTACTATCTCTTTTAATAATGCATAGGAATTCTTT 50
     ||||||||||||||||||||||||||||||||||||||||||||||||||
3365 CCAATACATATTTTATTACTATCTCTTTTAATAATGCATAGGAATTCTTT 3414
                .         .         .         .         .
  51 TTTAGACTAGTTTTTGGGTTTGCTTTGTCTATAACAAAAAATAAATACAA 100
     ||||||||||||||||||||||||||||||||||||||||||||||||||
3415 TTTAGACTAGTTTTTGGGTTTGCTTTGTCTATAACAAAAAATAAATACAA 3464
                .         .         .         .         .
 101 CAACTTAATAATCACATTTGAAAACAAATTTAACAAGTATAATGTGAGTT 150
     ||||||||||||||||||||||||||||||||||||||||||||||||||
3465 CAACTTAATAATCACATTTGAAAACAAATTTAACAAGTATAATGTGAGTT 3514
                .         .         .         .         .
 151 TTTCTTTTTCCTAACTTCCTCAGGACTTAGGTCACCTTTATTAAAAGGA 200
     |||||||||||||||||||||||||| |||||||||||||||||||||||
3515 TTTCTTTTTCCTAACTTCCTCAGGACCTAGGTCACCTTTATTAAAAGGA 3564
                .         .         .         .         .
 201 AGAATTCACTCTTTTTTCCTTGGCAGTTTAATCATTCAGGAATCCCTCTC 250
     ||||||||||||||||||||||||||||||||||||||||||||||||||
3565 AGAATTCACTCTTTTTTCCTTGGCAGTTTAATCATTCAGGAATCCCTCTC 3614
                .         .         .         .         .
 251 TTAAAGGAAATTGTTATTTTATTCATGTAACCTTTTTTTGTAATCAAAAG 300
     ||||||||||||||||||||||||||||||||||||||||||||||||||
3615 TTAAAGGAAATTGTTATTTTATTCATGTAACCTTTTTTTGTAATCAAAAG 3664
                .         .
 301 TGAATAAAAACGATCTTTTTGTCTTACA 328
     ||||||||||||||||||||||||||||
3665 TGAATAAAAACGATCTTTTTGTCTTACA 3692
```

**Fig. 8 Schematic alignment of SEQ ID NO. 3 with human Ensadin-0581 cDNA**

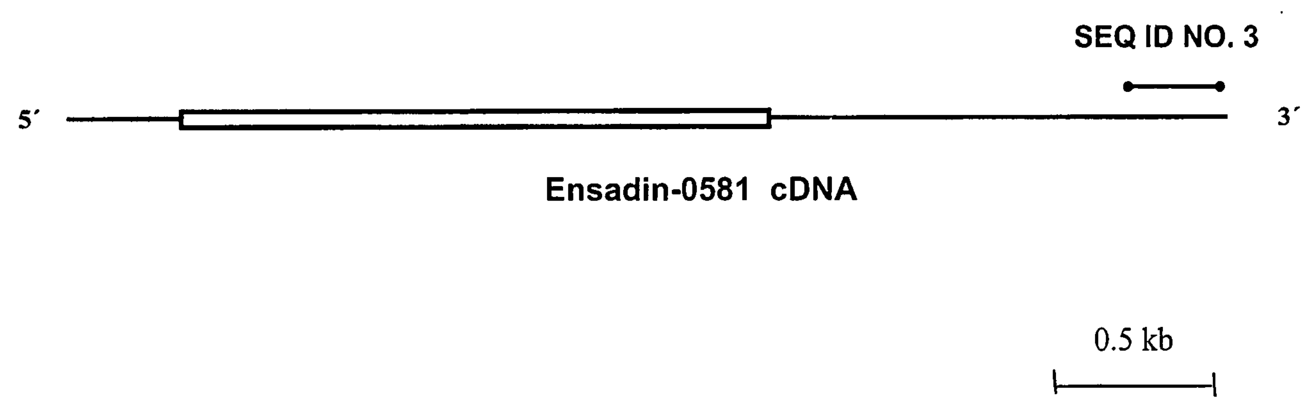

**Table 1: Identification of differentially expressed genes in microarray hybridization experiments**

| Biochip | Type of probe | Used probes (Cy5-/Cy3-labeled) | Ratio fluorescence intensity: temporal / frontal cortex |
|---|---|---|---|
| 1 | C | $PT_{SSH(2)}$ / $PF_{SSH(1)}$ | 1.78 |
| 2 | B | PT / PF | 1.58 |
| 3 | A | PT / PF | 1.94 |

**Table 2 :**

| sample | Δ (fold) (temporal / frontal cortex) |
|---|---|
| control C011 | 0.98 |
| control C012 | 1.01 |
| control C014 | 0.51 |
| control C005 | 1.02 |
| control C008 | 1.02 |
| patient P012 | 1.21 |
| patient P016 | 1.95 |
| patient P010 | 1.77 |
| patient P011 | 1.03 |
| patient P014 | 1.95 |
| patient P017 | 1.01 |
| patient P019 | 1.76 |

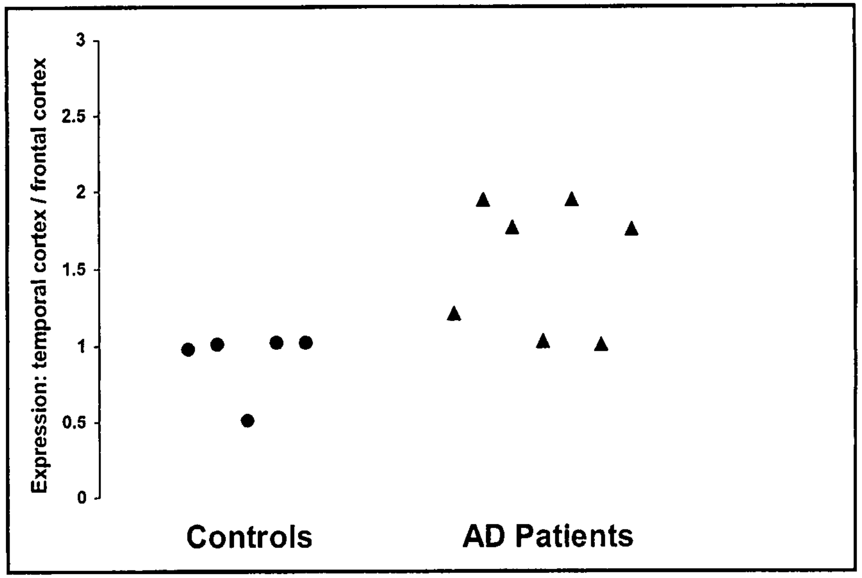

**Table 3:**

| sample | Δ (fold) (hippocampus / frontal cortex) |
|---|---|
| control C005 | 0.81 |
| control C008 | 1.09 |
| control C004 | 2.04 |
| patient P012 | 1.08 |
| patient P016 | 2.41 |
| patient P010 | 1.57 |
| patient P011 | 1.23 |
| patient P014 | 0.99 |
| patient P019 | 2.13 |

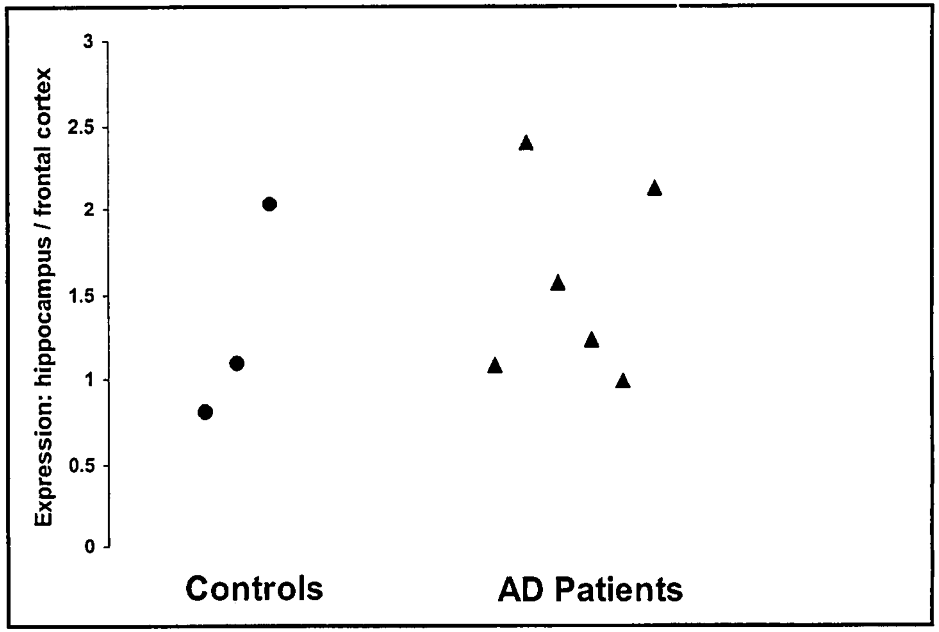

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A
- WO 02014313 A


### Non-patent literature cited in the description


- **VICKERS et al.** *Progress in Neurobiology,* 2000, vol. 60, 139-165
- **SELKOE.** *Physiological Rev,* 2001, vol. 81, 741-66
- **GREENFIELD et al.** *Frontiers Bioscience,* 2000, vol. 5
- **BRAAK ; BRAAK.** *Acta Neuropathol,* 1991, vol. 82, 239-259
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** *Annals of Neurology,* 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **MYERS et al.** *Science,* 2000, vol. 290, 2304-5
- **BERTRAM et al.** *Science,* 2000, vol. 290, 2303
- **SCOTT et al.** *Am J Hum Genet,* 2000, vol. 66, 922-32
- **NAGASE et al.** *DNA Research,* 2001, vol. 8, 179-187
- **HE.** *Int Rev Cytol,* 1995, vol. 162B, 1-74
- **COLLINS et al.** *Molecular and Cellular Biology,* 2001, vol. 21, 3609-3615
- **ADAMS et al.** *Trends in Cell Biology,* 2000, vol. 10, 17-24
- **XUE ; COOLEY.** *Cell,* 1993, vol. 72, 681-693
- **ROBINSON ; COOLEY.** *Journal of Cell Biology,* 1997, vol. 138, 799-810
- **NEMES et al.** *Human Molecular Genetics,* 2000, vol. 9, 1543-1551
- **KIM et al.** *Journal of Cell Biology,* 1998, vol. 141, 553-566
- **SOLTYSIK-ESPANOLA et al.** *Mol Biol Cell,* 1999, vol. 10, 2361-2375
- **CHEN et al.** *Journal of Biological Chemistry,* 2002
- **IQBAL ; SWAAB ; WINBLAD ; WISNIEWSKI.** Alzheimer's Disease and Related Disorders (Etiology, Pathogenesis and Therapeutics). Wiley & Sons, 1999
- **SCINTO ; DAFFNER.** Early Diagnosis of Alzheimer's Disease,. Humana Press
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **SCHENA M.** *Microarray Biochip Technology,* 2000
- **CAPECCHI.** *Science,* 1989, vol. 244, 1288-1292
- **HOGAN et al.** *Manipulating the Mouse Embryo: A Laboratory Manual,* 1994
- **JACKSON ; ABBOTT.** *Mouse Genetics and Transgenics: A Practical Approach,* 1999
- **SCHWARZ et al.** *Biochemistry,* 1999, vol. 38, 9456-9464

- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **TERRY et al.** *Annals of Neurology,* 1981, vol. 10, 184-192
- **STRANGE.** Brain Biochemistry and Brain Disorders. Oxford University Press, 1992, 4
- Gene Cloning and Analysis by RT-PCR. **CHENCHIK et al.** Biotechniques Books. 1998, 305-320

SEQUENCE LISTING

<110> EVOTEC NeuroSciences GmbH

<120> Diagnostic and therapeutic use of ensadin-0581 gene and protein for neurodegenerative diseases

<130> 021757 ep ME/BM

<140> 02014313.7
<141> 2002-06-27
<160> 15

<170> PatentIn Ver. 2.1

<210> 1
<211> 619
<212> PRT
<213> Homo sapiens

<400> 1

```
Met Pro Ser Glu Arg Cys Leu Ser Ile Gln Glu Met Leu Thr Gly Gln
 1               5                  10                  15

Arg Leu Cys His Ser Glu Ser His Asn Asp Ser Val Leu Ala Ala Leu
            20                  25                  30

Asn Gln Gln Arg Ser Asp Gly Ile Leu Cys Asp Ile Thr Leu Ile Ala
        35                  40                  45

Glu Glu Gln Lys Phe His Ala His Lys Ala Val Leu Ala Ala Cys Ser
    50                  55                  60

Asp Tyr Phe Arg Ala Met Phe Ser Leu Cys Met Val Glu Ser Gly Ala
65                  70                  75                  80

Asp Glu Val Asn Leu His Gly Val Thr Ser Leu Gly Leu Lys Gln Ala
                85                  90                  95

Leu Glu Phe Ala Tyr Thr Gly Gln Ile Leu Leu Glu Pro Gly Val Ile
            100                 105                 110

Gln Asp Val Leu Ala Ala Gly Ser His Leu Gln Leu Leu Glu Leu Leu
        115                 120                 125

Asn Leu Cys Ser His Tyr Leu Ile Gln Glu Leu Asn Ser Phe Asn Tyr
    130                 135                 140

Leu Asp Leu Tyr Arg Leu Ala Asp Leu Phe Asn Leu Thr Leu Leu Glu
145                 150                 155                 160

Lys Ala Val Ile Asp Phe Leu Val Lys His Leu Ser Glu Leu Leu Lys
                165                 170                 175

Ser Arg Pro Glu Glu Val Leu Thr Leu Pro Tyr Cys Leu Leu Gln Glu
            180                 185                 190

Val Leu Lys Ser Asp Arg Leu Thr Ser Leu Ser Glu Glu Gln Ile Trp
        195                 200                 205

Gln Leu Ala Val Arg Trp Leu Glu His Asn Cys His Tyr Gln Tyr Met
    210                 215                 220

Asp Glu Leu Leu Gln Tyr Ile Arg Phe Gly Leu Met Asp Val Asp Thr
225                 230                 235                 240

Leu His Thr Val Ala Leu Ser His Pro Leu Val Gln Ala Ser Glu Thr
```

245 250 255

Ala Thr Ala Leu Val Asn Glu Ala Leu Glu Tyr His Gln Ser Ile Tyr
260 265 270

Ala Gln Pro Val Trp Gln Thr Arg Arg Thr Lys Pro Arg Phe Gln Ser
275 280 285

Asp Thr Leu Tyr Ile Ile Gly Gly Lys Lys Arg Glu Val Cys Lys Val
290 295 300

Lys Glu Leu Arg Tyr Phe Asn Pro Val Asp Gln Glu Asn Ala Leu Ile
305 310 315 320

Ala Ala Ile Ala Asn Trp Ser Glu Leu Ala Ser Met Pro Val Gly Arg
325 330 335

Ser His His Cys Val Ala Val Met Gly Asp Phe Leu Phe Val Ala Gly
340 345 350

Gly Glu Val Glu His Ala Ser Gly Arg Thr Cys Ala Val Arg Thr Ala
355 360 365

Cys Arg Tyr Asp Pro Arg Ser Asn Ser Trp Ala Glu Ile Ala Pro Met
370 375 380

Lys Asn Cys Arg Glu His Phe Val Leu Gly Ala Met Glu Glu Tyr Leu
385 390 395 400

Tyr Ala Val Gly Gly Arg Asn Glu Leu Arg Gln Val Leu Pro Thr Val
405 410 415

Glu Arg Tyr Cys Pro Lys Lys Asn Lys Trp Thr Phe Val Gln Ser Phe
420 425 430

Asp Arg Ser Leu Ser Cys His Ala Gly Tyr Val Ala Asp Gly Leu Leu
435 440 445

Trp Ile Ser Gly Gly Val Thr Asn Thr Ala Gln Tyr Gln Asn Arg Leu
450 455 460

Met Val Tyr Glu Pro Asn Gln Asn Lys Trp Ile Ser Arg Ser Pro Met
465 470 475 480

Leu Gln Arg Arg Val Tyr His Ser Met Ala Val Gln Arg Lys Leu Tyr
485 490 495

Val Leu Gly Gly Asn Asp Leu Asp Tyr Asn Asn Asp Arg Ile Leu Val
500 505 510

Arg His Ile Asp Ser Tyr Asn Ile Asp Thr Asp Gln Trp Thr Arg Cys
515 520 525

Asn Phe Asn Leu Leu Thr Gly Gln Asn Glu Ser Gly Val Ala Val His
530 535 540

Asn Gly Arg Ile Tyr Leu Val Gly Gly Tyr Ser Ile Trp Thr Asn Glu
545 550 555 560

Pro Leu Ala Cys Ile Gln Val Leu Asp Val Ser Arg Glu Gly Lys Glu
565 570 575

Glu Val Phe Tyr Gly Pro Thr Leu Pro Phe Ala Ser Asn Gly Ile Ala
580 585 590

Ala Cys Phe Leu Pro Ala Pro Tyr Phe Thr Cys Pro Asn Leu Gln Thr

595 600 605

Leu Gln Val Pro His His Arg Ile Gly Thr Ile
610 615

<210> 2
<211> 3693
<212> DNA
<213> Homo sapiens

<400> 2

attttttacta gggagcagtt tccccgcgcg acagttcggg agcgcgcagg cagtcgcgcg 60
cacacacgca cgcaggcaca cacacacaca cacacacaca cactttcgca cacacaaaca 120
cgctaggacg ctcgtcttcg ctactgcatc cccgaaccag cagagcgaag ctactgcggg 180
ttctgttaac ctcagcatcg tggggcgaag cagagccatt gtgcatcaag gagaggccgg 240
tgcctgcgct gccgtctctg gcacctaacc cagcagaccg ctcaccccat cggctggaat 300
gcttgctgat tcctctgcac acttctaagg ctgagaacct gaggaaccca gctggaaaat 360
gccgtctgaa cgctgcctca gtattcaaga aatgctgaca ggccagaggc tctgccactc 420
cgaatctcac aatgacagtg tcctggcagc gctgaatcag cagaggagtg atggcatcct 480
ctgcgacatc accctgattg ctgaggaaca gaaattccat gctcacaagg cagtcctagc 540
agcatgcagt gactatttcc gggcaatgtt cagtctttgt atggtggaaa gtggagctga 600
tgaggttaat ttgcacggtg tgaccagcct tggcttaaag caggctctgg agtttgcata 660
cacaggacag attttgctgg agccaggtgt gatccaggat gtgctagcag cgggcagtca 720
cctacagctg ttggagcttc tcaatttatg ctcccactat ctcatccagg aattaaatag 780
ctttaattac ttggatctgt acagacttgc tgacctcttt aacctcactt tgttggagaa 840
ggcagtgatc gatttcttag tgaaacatct ctctgaactc ctgaagagcc gcccagaaga 900
agttctaacg cttccctatt gcctgcttca ggaggtgctg aagagcgacc gcctgacctc 960
cctgagtgaa gagcagatct ggcagctagc tgtgaggtgg ttggaacaca actgccacta 1020
ccagtacatg gacgagctcc tgcaatacat ccgctttggc ctaatggatg tggatactct 1080
ccatacagtt gccctgtccc accccttgt ccaagcaagt gagactgcaa cagcccttgt 1140
caacgaggcc ctggaatacc accagagcat ctatgcacag cctgtctggc agactcgcag 1200
gaccaaacca cgattccagt cagacactct gtatatcatt ggtgggaaaa agcgcgaggt 1260
ctgcaaggtc aaggaacttc ggtacttcaa tcctgttgat caggagaatg ctctcatagc 1320
tgccattgcc aactggagtg agctggctcc catgcctgtg ggaaggagcc accattgtgt 1380
ggcagtcatg gggacttcc tgtttgtggc aggaggggaa gttgagcatg ccagtggccg 1440
gacgtgtgct gtgaggactg cctgtcgcta tgacccccgc agtaattcct gggcagagat 1500
agcacccatg aaaaactgcc gggagcattt tgtgctgggt gccatggagg aatacctcta 1560
tgcagttggg ggcagaaatg aactgcgcca ggttctgcct acagttgagc gatattgccc 1620
caagaagaac aaatggactt ttgttcagtc ctttgacaga tccctttcat gccatgctgg 1680
atatgtggct gatggtcttc tttggatatc aggtggagta actaatacgg cacaatatca 1740
gaacaggcta atggtgtatg aacctaacca gaataagtgg ataagccgta gccccatgct 1800
gcagagaagg gtctaccatt ccatggctgc tgtacaaagg aagctttatg ttcttggagg 1860
caatgaccta gactacaata atgaccggat ccttgtgcgc catatagatt cttacaacat 1920
agacactgac cagtggacac gttgtaattt caacctgctg actggccaaa atgaatctgg 1980
agttgctgtc cataatggga gaatatattt agttggtgga tattcaattt ggacaaatga 2040
gcctctggct tgtatccagg tactggatgt aagcagagaa ggcaaagaag aagtattcta 2100
tgggcctaca ctcccttttg cttccaatgg aatagcagca tgcttccttc cagctccata 2160
ttttacatgc cctaaccttc aaactcttca agtgcctcat cacaggattg gcaccatctg 2220
aaaagccaag ccatcatgaa caggaggaaa acatagctct gactgttgga tactgggcat 2280
gaaaagactc agtgctccat gcttccttgt cttgctttat aggtcttata ttcggataaa 2340
tttaagcaaa aaatgaacaa ttttctaaaa tacgttattg aaaactcgtc acccttctca 2400
gtgtatgtca acattcaata tgtatgactt ttattgtggt atagcttagt gccaatttaa 2460
ggtatattgt gttccatggg tctttagagc attctttgta cactttttct aatcagcact 2520
gtcttaagac aacataacac tgttagtaag gcaatttatt ggacacaatg gcatcgatga 2580
tcttaaaaat atatatattc tgtacttaat ccctttatta tttaaagccg ggcttgtaat 2640
ttttcttttt aaaatttaac attatgcagc cctgctcagt agagactcac tcagcattat 2700
gacatcataa atttttaaat gccatatttt attcaaggtg atacgaataa taatagacat 2760
acactggaac agcagttgtt gaaataccca gatttttcac cttggttact ctgaccttgg 2820
gtcagaacct aactttacag tgcttcagtt ttcccacctt ttaaggagga taattactga 2880
tttccacgtt aatttaaaag aacattatga ggattaggcg aacactttgt aatactttgc 2940
ccaagaaaaa gaaaaatgtt gttactgaaa ttataaggta ttttattgtc tttagaacct 3000
tgtaattgaa cagggagatt atttttgaag tttaaaaatt actagataga atttggaaac 3060
atgttgtgga ataaatgatt ctaaaaagca tgtgaaagag acacacaatg aggaaatagg 3120
gagtttctca aaatctaaac ttgtacaggt atctaaactt gcctcaagta actctcagat 3180
gtcttattta aaatcattta gtgaaaaatg acatttaaag ctaaaaaata atttagttgt 3240
actaattatg gtaccataaa gtgctttgac ataaatttga acctagaatt ggcagttcta 3300

```
ataaaagttt ttccactttg ttaaaggtta tgtcaatctt gagtgcttgt ggaattcttc 3360
accaccaata catattttat tactatctct tttaataatg cataggaatt ctttttttaga 3420
ctagtttttg ggtttgcttt gtctataaca aaaaataaat acaacaactt aataatcaca 3480
tttgaaaaca aatttaacaa gtataatgtg agtttttctt ttttcctaac ttcctcagga 3540
cctaggtcac ctttattaaa aggaagaatt cactcttttt tccttggcag tttaatcatt 3600
caggaatccc tctcttaaag gaaattgtta ttttattcat gtaacctttt tttgtaatca 3660
aaagtgaata aaaacgatct ttttgtctta cag                              3693
```

<210> 3
<211> 336
<212> DNA
<213> Artificial Sequence
<220>
<223> Description of Artificial Sequence: cDNA fragment of ensadin-0581

<400> 3

```
sdnngthbcc aatacatatt ttattactat ctcttttaat aatgcatagg aattcttttt 60
tagactagtt tttgggtttg ctttgtctat aacaaaaaat aaatacaaca acttaataat 120
cacatttgaa aacaaattta acaagtataa tgtgagtttt tctttttttcc taacttcctc 180
aggacttagg tcacctttat taaaaggaag aattcactct tttttccttg gcagtttaat 240
cattcaggaa tccctctctt aaaggaaatt gttattttat tcatgtaacc tttttttgta 300
atcaaaagtg aataaaaacg atctttttgt cttaca                           336
```

<210> 4
<211> 20
<212> DNA
<213> Artificial Sequence
<220>
<223> Description of Artificial Sequence: primer for ensadin-0581

<400> 4
ggcaccatct gaaaagccaa 20

<210> 5
<211> 23
<212> DNA
<213> Artificial Sequence
<220>
<223> Description of Artificial Sequence: primer for ensadin-0581

<400> 5
cccagtatcc aacagtcaga gct 23

<210> 6
<211> 20
<212> DNA
<213> Artificial Sequence
<220>
<223> Description of Artificial Sequence: primer for cyclophilin B

<400> 6
actgaagcac tacgggcctg 20

<210> 7
<211> 19

<212> DNA
<213> Artificial Sequence
<220>
<223> Description of Artificial Sequence: primer for cyclophilin B

<400> 7
agccgttggt gtcctttgcc 19

<210> 8
<211> 20
<212> DNA
<213> Artificial Sequence
<220>
<223> Description of Artificial Sequence: primer for ribosomal protein S9

<400> 8
ggtcaaattt accctggcca 20

<210> 9
<211> 22
<212> DNA
<213> Artificial Sequence
<220>
<223> Description of Artificial Sequence: primer for ribosomal protein S9

<400> 9
tctcatcaag cgtcagcagt tc 22

<210> 10
<211> 19
<212> DNA
<213> Artificial Sequence
<220>
<223> Description of Artificial Sequence: primer for beta-actin

<400> 10
tggaacggtg aaggtgaca 19

<210> 11
<211> 19
<212> DNA
<213> Artificial Sequence
<220>
<223> Description of Artificial Sequence: primer for beta-actin

<400> 11
ggcaagggac ttcctgtaa 19

<210> 12
<211> 20
<212> DNA
<213> Artificial Sequence
<220>
<223> Description of Artificial Sequence: primer for GAPDH

<400> 12
cgtcatgggt gtgaaccatg 20

<210> 13
<211> 21
<212> DNA
<213> Artificial Sequence
<220>
<223> Description of Artificial Sequence: primer for GAPDH

<400> 13
gctaagcagt tggtggtgca g 21

<210> 14
<211> 21
<212> DNA
<213> Artificial Sequence
<220>
<223> Description of Artificial Sequence: primer for the transferrin receptor

<400> 14
gtcgctggtc agttcgtgat t 21

<210> 15
<211> 23
<212> DNA
<213> Artificial Sequence
<220>
<223> Description of Artificial Sequence: primer for the transferrin receptor

<400> 15
agcagttggc tgttgtacct ctc 23